(19) **Europäisches Patentamt European Patent Office Office européen des brevets**

(11) **EP 4 451 285 B1**

(12) **EUROPEAN PATENT SPECIFICATION**

(45) Date of publication and mention
of the grant of the patent:
**09.07.2025 Bulletin 2025/28**

(51) International Patent Classification (IPC):
**G16H 50/20** (2018.01)     **G16B 30/00** (2019.01)

(52) Cooperative Patent Classification (CPC):
**G16H 50/20; G16B 30/00**

(21) Application number: **24153673.9**

(22) Date of filing: **24.01.2024**

(54) **METHOD AND SYSTEM FOR IDENTIFYING AND UTILIZING FRUGAL MARKERS FOR CLASSIFICATION OF BIOLOGICAL SAMPLE**

VERFAHREN UND SYSTEM ZUR IDENTIFIZIERUNG UND VERWENDUNG VON FRUCTALMARKERN ZUR KLASSIFIZIERUNG BIOLOGISCHER PROBEN

PROCÉDÉ ET SYSTÈME D'IDENTIFICATION ET D'UTILISATION DE MARQUEURS FRUGAUX POUR LA CLASSIFICATION D'ÉCHANTILLON BIOLOGIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **19.04.2023 IN 202321028613**

(43) Date of publication of application:
**23.10.2024 Bulletin 2024/43**

(73) Proprietor: **Tata Consultancy Services Limited Maharashtra (IN)**

(72) Inventors:
• **HAQUE, MOHAMMED MONZOORUL**
  **411013 Pune, Maharashtra (IN)**
• **SINGH, RASHMI**
  **411013 Pune, Maharashtra (IN)**
• **MERCHANT, MITALI**
  **411013 Pune, Maharashtra (IN)**
• **MANDE, SHARMILA SHEKHAR**
  **411013 Pune, Maharashtra (IN)**
• **CHENNAREDDY, VENKATA SIVA KUMAR REDDY**
  **411013 Pune, Maharashtra (IN)**
• **NAGPAL, SUNIL**
  **110001 New Delhi, Delhi (IN)**
• **DUTTA, ANIRBAN**
  **411013 Pune, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**WO-A1-2023/034618**

• **DONG SITONG ET AL: "16S rDNA Full-Length Assembly Sequencing Technology Analysis of Intestinal Microbiome in Polycystic Ovary Syndrome", FRONTIERS IN CELLULAR INFECTION MICROBIOLOGY, vol. 11, 10 May 2021 (2021-05-10), CH, XP093179958, ISSN: 2235-2988, DOI: 10.3389/fcimb.2021.634981**
• **PARK SUNWHA ET AL: "Prediction of preterm birth based on machine learning using bacterial risk score in cervicovaginal fluid", AMERICAN JOURNAL OF REPRODUCTIVE IMMUNOLOGY, vol. 86, no. 3, 1 September 2021 (2021-09-01), US, XP093178782, ISSN: 1046-7408, DOI: 10.1111/aji.13435**
• **MARCOS-ZAMBRANO LAURA JUDITH ET AL: "Applications of Machine Learning in Human Microbiome Studies: A Review on Feature Selection, Biomarker Identification, Disease Prediction and Treatment", FRONTIERS IN MICROBIOLOGY, vol. 12, 19 February 2021 (2021-02-19), XP055834142, DOI: 10.3389/fmicb.2021.634511**

**Description**

CROSS-REFERENCE TO RELATED APPLICATIONS AND PRIORITY

The present application claims priority to Indian application no. 202321028613, filed on April 19, 2023.

TECHNICAL FIELD

The disclosure herein generally relates to the field of a biomarker identification and, more particularly, to a method and system for identifying and utilizing frugal set of markers for classification of a test biological sample.

BACKGROUND

Search for novel biomarkers to diagnose the occurrence or progression of non-communicable disease or assessing therapeutic response (patient stratification), is an active field of research. Technological advances in the fields of microfluidics and imaging, applied to the domain of biochemistry and life sciences, has enabled high throughput screening of multiple biochemicals, metabolites, gene-expressions, oligonucleotides etc. from a biological/ environmental sample, using fewer experiments. These advances have therefore driven research towards search for diagnostic solutions that can rely on multiple biomarkers for a disease diagnosis, given that multiple biomarkers in combination are expected to provide more accurate results than diagnostic solutions relying on single biomarkers.

Many next-generation IVD (in-vitro diagnostic) solutions employing this strategy have enabled assessment of diseases/ disorders which would otherwise remain undetected. However, discovering an optimal and/ or frugal set of features/ biomarkers form a large set of features measured through high-throughput screening techniques, which can characterize a disease/ anomaly with sufficient accuracy, remains a challenge. DONG SITONG ET AL: "16S rDNA Full-Length Assembly Sequencing Technology Analysis of Intestinal Microbiome in Polycystic Ovary Syndrome", FRONTIERS IN CELLULAR INFECTION MICROBIOLOGY, Dong. et. al study the characteristics and relationship of the gut microbiota in patients with polycystic ovary syndrome (PCOS). For this study, 45 patients recruited with PCOS and 37 healthy women from the Reproductive Department of Shengjing Hospital and recorded their clinical indexes, and sequenced their fecal samples by 16S rDNA full-length assembly sequencing technology (16S-FAST). The study found decreased $\alpha$ diversity and different abundances of a series of microbial species in patients with PCOS compared to healthy controls. The study found LH and AMH were significantly increased in PCOS with Prevotella enterotype when compared to control women with Prevotella enterotype, while glucose and lipid metabolism level remained no significant difference, and situations were opposite in PCOS and control women with Bacteroides enterotype. Ruminococcus gnavus, Prevotella stercorea, Dialister succinatiphilus and Bacteroides fragilis were more abundant while Christensenellaceae spp. were less abundant in the PCOS group. P. stercorea was significantly more prevalent in PCOS-not insulin resistance (NIR) compared to control-NIR and PCOS-not overweight (NOW) patient groups compared to control-NOW groups. Kyoto Encyclopedia Genes and Genomes reflecting pathways related to lipopolysaccharide biosynthesis were more abundant in the PCOS group.

SUMMARY

Embodiments of the present disclosure present technological improvements as solutions to one or more of the above-mentioned technical problems recognized by the inventors in conventional systems. The invention is set out in the appended set of claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates a network diagram of a system for identifying and utilizing a frugal set of markers for classification of a test biological sample, according to some embodiments of the present disclosure.

FIG. 2A-2B are flowcharts illustrating a method for identifying and utilizing a frugal set of markers for classification of a test biological sample, according to some embodiments of the present disclosure.

FIG. 3A-3C are flowcharts illustrating the steps involved in building an ensemble classification model, according to some embodiments of the present disclosure.

FIG. 4 illustrates an exemplary multiplex qPCR design for detecting and quantifying microbial DNA having an exemplary set of qPCR-probes, according to some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The existing methodologies for biomarker identification rely on different statistical methods as well as machine learning approaches, often ends up with classification models which are highly accurate but dependent on a significant higher number of features. On the other hand, classification models obtained using lesser number of features are often not accurate enough for clinical deployment. Although high-throughput screening methods can be used in biomarker discovery (and hypothesis testing) phases for screening/ measuring a large number of features (and making observational inferences from measurements corresponding to the same), higher costs prevent deployment of the same for diagnostics at a population scale. In effect, the discovered (set of) biomarker(s) find clinical relevance only with sufficient accuracy of prediction and can be measured economically. Ideally, a diagnostic method should rely on a small number of biomarkers/ features, which can be measured economically, and still provide sufficient accuracy.

Similarly, from the therapeutic perspective, biomarkers discovered assumes importance in cases where causal relationship of the biomarkers with the disease in question can be identified. Corrective measures to modulate the levels of the biomarkers can then be considered for effective disease management. Even in this scenario, identification of a smaller number of features (biomarkers) aid in practical therapeutic design.

According to an embodiment of the disclosure, a method provides a technical solution to the above-mentioned technical problems by identifying and utilizing a frugal set of markers for classification of a test biological sample.

The present disclosure provides a method for identification of a frugal combination of disease biomarkers that can enable accurate and easy assessment of a disease and guide in therapy. To be specific, given a set of measurements of multiple (physical/ biochemical/ genetic/ microbiological etc.,) features characterizing biological samples obtained from cohorts (a group of individuals) pertaining to disease cases and (healthy) controls, the present disclosure computes a metric (akin to a mathematical model) combining the measured values of a small subset (not exceeding 15) of the features (predetermined or preidentified), which can be used for classifying between case and control samples. Any newly obtained sample can be classified into the case or control group by using the defined metric, the computation of which only involves obtaining measured features corresponding to the pre-defined small subset (predetermined or preidentified). In effect, the method allows accurate diagnosis while using lesser (or frugal) number of features (implying lower cost of measurement), compared to methods taught by current state-of-art. By virtue of selecting a small set of features/ descriptors which are characteristic of the disease, the method further enables an easy trace-back towards causality and guides in recommending or designing a focused and personalized therapeutic design. Relying on the "guilt by association" principle, the therapeutic design may involve correcting the anomalous abundance patterns of the identified subset of features either by directly spiking or antagonizing them or by modulating the abundance levels of their close correlates (features).

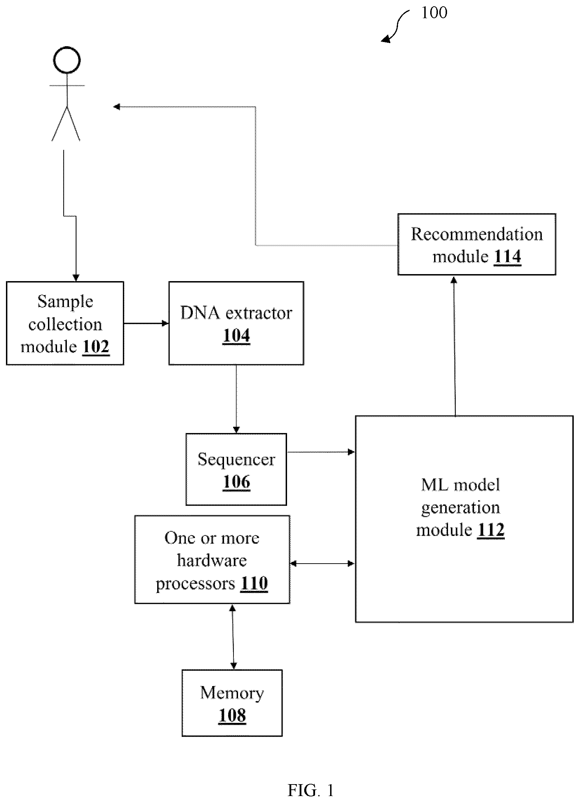

Referring now to the drawings, and more particularly to FIG. 1 through FIG. 4, where similar reference characters denote corresponding features consistently throughout the figures, there are shown preferred embodiments and these embodiments are described in the context of the following exemplary system and/or method.

According to an embodiment of the disclosure, a block diagram of the system 100 for identifying and utilizing a frugal set of markers for classification of a test biological sample is shown in FIG. 1. The system 100 consists of a sample collection module 102, a DNA extractor 104, a sequencer 106, a memory 108, one or more hardware processors (referred as a processor, herein after) 110, a machine learning model generation module 112, and a recommendation module 114 as shown in FIG. 1. The processor 110 is in communication with the memory 108.

It may be understood that the system 100 comprises one or more computing devices, such as a laptop computer, a desktop computer, a notebook, a workstation, a cloud-based computing environment and the like. It will be understood that the system 100 may be accessed through one or more input/output interfaces (not shown in Figures), collectively referred to as I/O interface or user interface. Examples of the I/O interface may include, but are not limited to, a user interface, a portable computer, a personal digital assistant, a handheld device, a smartphone, a tablet computer, a workstation and the like. The I/O interface are communicatively coupled to the system 100 through a network.

In an embodiment, the network may be a wireless or a wired network, or a combination thereof. In an example, the network can be implemented as a computer network, as one of the different types of networks, such as virtual private network (VPN), intranet, local area network (LAN), wide area network (WAN), the internet, and such. The network may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network may include a variety of network devices, including routers, bridges, servers, computing devices, storage devices. The network devices within the network may interact with the system 100 through communication links.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

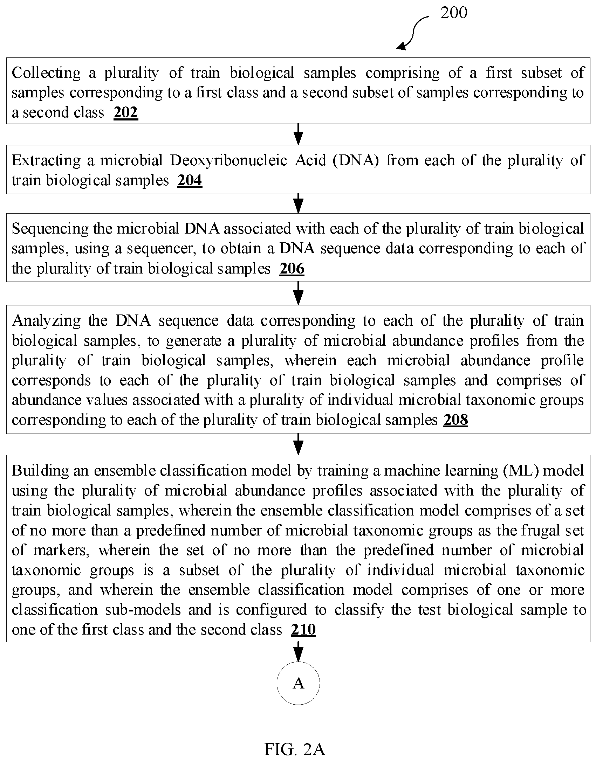

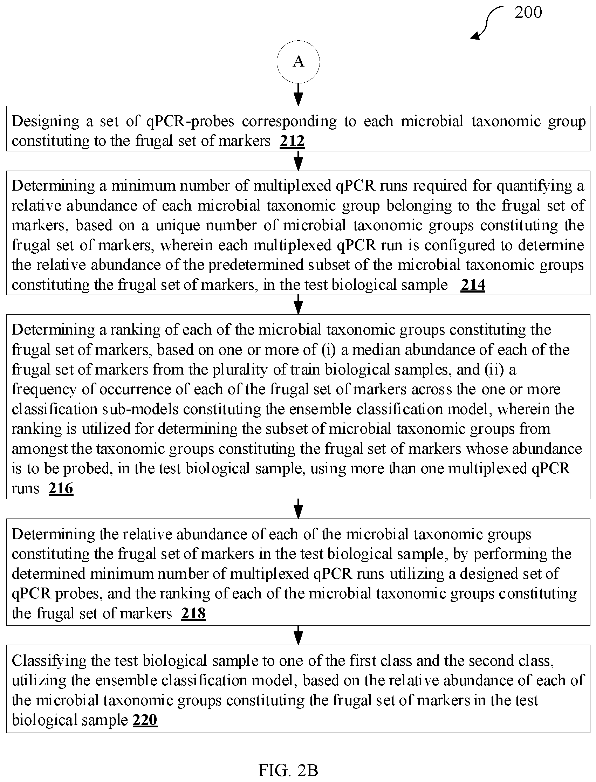

In operation, a flow diagram of a method 200 for identifying and utilizing a frugal set of markers for classification of a test biological sample, according to some embodiments of the present disclosure is shown in FIG. 2A-2B. The method 200 depicted in the flow chart may be executed by a system, for example, the system, 100 of FIG. 1. In an example embodiment, the system 100 may be embodied in a computing device.

Operations of the flowchart, and combinations of operation in the flowchart, may be implemented by various means, such as hardware, firmware, processor, circuitry and/or other device associated with execution of software including one or more computer program instructions. For example, one or more of the procedures described in various embodiments may be embodied by computer program instructions. In an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 200 are described with help of system 100. However, the operations of the method 200 can be described and/or practiced by using any other system.

Initially at step 202, the method 200 a plurality of train biological samples is collected through the sample collection module 102. Each train biological sample comprises at least one of a vaginal swab sample, a cervical mucus sample, a cervical swab sample, a vaginal swab including swab sample of a vaginal fornix, a urine sample, an amniotic fluid sample, a blood sample (whole blood sample), a serum sample, a plasma sample, a placental swab, an umbilical swab, a stool sample, a skin swab, an oral swab, a saliva sample, a periodontal swab, a throat swab, a nasal swab, a vesicle fluid sample, a nasopharyngeal swab, a nares swab, a conjunctival swab, a genital swab, a rectum swab, a tracheal aspirate, and a bronchial swab. The plurality of train biological sample comprises of a subset of samples corresponding to a first class and a remaining subset of samples corresponding to a second class. The biological samples refer to any type of samples sourced from animals, humans, plants etc. The first class and the second class refer to two different biological states that are exhibited by the individual/ environment from where the sample is sourced/ collected. For example, the first class may refer to an individual (or a group of individuals) in a healthy class and the second class may refer to an individual (or a group of individuals) in a diseased class.

Further at step 204, A microbial Deoxyribonucleic Acid (DNA) is extracted from each of the plurality of train biological samples, through the DNA extractor 104. At step 206, the extracted microbial DNA associated with each train biological sample is then sequenced through the sequencer 106 to obtain a DNA sequence data. The sequencer 106 amplifies and sequences either full-length or specific variable regions of the bacterial 16S rRNA or any other marker genes from the extracted microbial DNA, or any appropriate signature-/ marker-gene sequences from the extracted microbial DNA, using a next-generation DNA sequencing (NGS) platform, or Oxford nanopore sequencing, or any other DNA sequencing technique and platform (including classical Sanger sequencing). In another implementation, alternate ways of characterizing/ sequencing/measuring parameters of the train biological sample, such as whole genomics shotgun (WGS) metagenomic sequencing, proteomics, metabolomics, etc., can also be used.

At step 208 of the method 200, the DNA sequence data associated with each train biological sample is then analyzed to generate a microbial abundance profile. A plurality of microbial abundance profiles is generated from the plurality of train biological samples. Each microbial abundance profile associated with each train biological sample comprise abundance values associated with a plurality of individual microbial taxonomic groups present in each of the plurality of train biological samples.

At step 210 of the method 200, an ensemble classification model is built using the microbial abundance profiles generated from the plurality of train biological samples, through the ML model generation module 112. The ensemble classification model comprises of a set of no more than a predefined number of microbial taxonomic groups as the frugal set of markers. The set of no more than the predefined number of microbial taxonomic groups is a subset of plurality of individual microbial taxonomic groups. Further, the ensemble classification model comprises of one or more classification sub-models and is configured to classify a test biological sample to one of the first class and the second class.

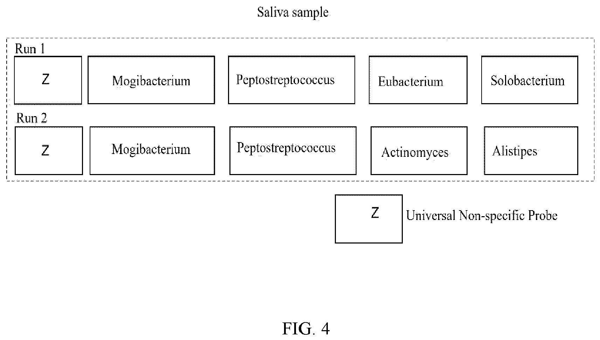

Further at step 212 of the method 200, a set of qPCR-probes corresponding to the microbial taxonomic groups constituting the frugal set of markers, is designed. FIG. 4 illustrates an exemplary multiplex qPCR design for detecting and quantifying microbial DNA having an exemplary set of qPCR-probes, according to some embodiments of the present disclosure. As shown in FIG. 4, the two sequential multiplex qPCR runs namely a first multiplex qPCR run (Run 1) and a second multiplex qPCR run (Run 2) are defined/performed for determining quantitative abundance of each of the microbial taxonomic groups (belonging to the frugal set of markers), whose DNA is targeted by the designed set of qPCR-probes. Each run of the first multiplex qPCR run (Run 1) and the second multiplex qPCR run (Run 2) includes five probes (hence it is also called as five-plex qPCR run) where each probe is utilized for detecting corresponding microbial taxonomic groups (constituting the frugal set of markers) each of the set of qPCR-probes, from the list having: *Mogibacterium, Peptostreptococcus, Eubacterium, Solobacterium, Actinomyces,* and *Alistipe.* Also, each run of the first multiplex qPCR run (Run 1) and the second multiplex qPCR run (Run 2) contains a universal non-specific probe (denoted as 'Z' in FIG. 4).

**Design configuration & number of qPCR runs required for quantifying the abundance of target microbial taxa/ features:**

The relative abundance of each of the microbial taxonomic groups constituting the frugal set of markers, that are common to each of the minimum number of the multiplexed qPCR runs, is determined based on a normalizing factor ($NF_{run}$) associated with each multiplexed qPCR run and the relative abundance of associated microbial taxonomic group in the corresponding multiplexed qPCR run.

Let us assume that a maximum of five unique DNA fragments, each representing a microbial taxa or spike DNA, can be quantified in a one multiplexed qPCR run. Therefore, to analyze a disease signature (captured in an ML model) comprising of '$n$' microbial taxa/ features, a minimum of (1 + [(n - 4)/4]) multiplexed qPCR runs would be required wherein '$n$' is the unique number of microbial taxonomic groups constituting the frugal set of markers, and wherein each multiplexed qPCR run is configured to determine, in the test biological sample, the relative abundance of a predetermined subset of the microbial taxonomic groups constituting the disease signature. This minimum number is based on assumptions that:

(a) the spike DNA should be analyzed at least once in one of the '(1 + [(n - 4)/41)' multiplexed qPCR runs; and
(b) an overlap of at least one microbial taxa/ features was done between two corresponding runs.

For example, if a disease signature comprises of 8 microbial taxa (A, B, C, D, E, F, G, and H), then at least TWO multiplexed qPCR runs would be required, where Z is the spike DNA of known concentration and taxa 'D' is analyzed in both multiplexed qPCR runs. Here, $\lceil (n - 4)/4 \rceil$ indicates a ceiling value of the expression. Thus, the minimum no. of required qPCR runs would be:

1 for 1-4 signatures/ features
2 for 5-8 signatures/ features
3 for 9-12 signatures/ features
4 for 13-16 signatures/ features, and so on...

**Example A: Run 1:** Z A B C <u>D</u>**; Run2: <u>D</u>** E F G H

Similarly, for a feature size of 12 (A, B, C, D, E, F, G, H, I, J, K, and L), at least THREE multiplexed qPCR runs would be required, where Z is the spike DNA of known concentration and taxa 'D' and 'H' are analyzed in twice.

**Example B: Run 1: Z** A B C <u>D</u>**; Run 2: <u>D</u>** E F G <u>H</u>**; Run 3: <u>H</u>** I J K L

If the number of features constituting the signature is not optimal for the above condition, i.e., for e.g., the number of features is 10, then more than one microbial taxon can be analyzed twice. The same is exemplified below, wherein taxa C and D are analyzed twice (in Runs 1 and 2). Similarly, taxa F and G are also analyzed twice (in Runs 2 and 3).

**Example C: Run 1:** Z A B <u>C D</u>**; Run 2: <u>C D</u>** E <u>F G</u>**; Run3: <u>F G</u>** H I J

In alternate implementations, the spike DNA (Z) can be analyzed in each of the runs. In that scenario, the first multiplexed qPCR will be able to accommodate up to FOUR features. Each additional multiplexed qPCR run will accommodate up to THREE new/ additional features as shown by underlining in the example below. Thus, two multiplexed qPCR runs would be required for a feature set of up to seven; three qPCR runs for a feature set of up to ten and so on.

**Run 1: Z** A B C <u>D</u> **; Run 2: Z** D <u>E F</u> <u>G</u> **; Run 3: Z** G <u>H I J</u>

Furthermore, if the number of features is not optimal for the above condition, then two or more taxa/ features can be analyzed multiple times as shown in example C.

**Methodology to interpret/ quantify the abundance of a microbial taxon from data obtained from above qPCR configurations:** Given that the concentration of the spike DNA (Z) is previously known - say $X_1$. If the measured concentration of Z in the multiplexed qPCR is $X_2$, then all the measured concentration in a single multiplexed qPCR run can be normalized multiplying by a normalizing factor ($NF_{run}$) of $X_1/X_2$.

In cases where the spike DNA is only analyzed in only one of the multiplexed qPCR runs (as shown in examples A, B and C), then the normalized values of the taxa/ feature in the first run which is/ are re-analyzed in the Run 2, can be used for adjusting the concentrations inferred from the Run 2 of the multiplexed qPCR. Following Example-A (described previously),

Actual conc of Z: $X_1$
Measured conc of Z: $X_2$
Normalizing factor $NF_{run1}$: $X_1/X_2$
Inferred conc. of A (from Run 1): $A'_{run1} \times NF_{run1}$
Inferred conc. of B (from Run 1): $B'_{run1} \times NF_{run1}$
Inferred conc. of C (from Run 1): $C'_{run1} \times NF_{run1}$
Inferred conc. of D (from Run 1): $D'_{run1} \times NF_{run1}$

Where $A'_{run1}$, $B'_{run1}$, $C'_{run1}$, and $D'_{run1}$ are the measured/ analyzed concentrations of taxa/ feature A, B, C and D respectively.

Normalizing factor $NF_{run2}$: Inferred conc. of D from Run 1/ Measured concentrations of feature D in Run 2

Inferred conc. of E: $E'_{run1} \times NF_{run2}$
Inferred conc. of F: $F'_{run1} \times NF_{run2}$
Inferred conc. of G: $G'_{run1} \times NF_{run2}$
Inferred conc. of H: $H'_{run1} \times NF_{run2}$

The same protocol may be repeated for normalizing/ adjusting the concentrations measured from all subsequent runs (as in example B). In case wherein more than once feature is analyzed in subsequent runs (as in example C), a median Normalizing factor ($NF$) - derived from the $NF$s for each of the replication features may be used for computing the inferred concentrations from that run.

In alternate implementations, wherein the spike DNA (Z) is analyzed in each of the runs (as in example D), Normalizing factor ($NF$) corresponding to each of the runs may be computed and used for inferring the concentrations of the constituent features. In cases, where the measured spike DNA (Z) concentration varies by more than 25% from the actual concentration, it is suggested that the observations from the said multiplexed qPCR run be discarded, and a fresh multiplexed qPCR run for the sub-set of features be performed.

In an alternate implementation using multiplex qPCR runs, the marker feature having the lowest variance in relative abundance in training data across both the classes, is selected as the anchor marker ($AM$), and the relative abundance of each of the markers is computed by multiplying the ratio of their estimated/inferred DNA concentrations and the estimated/inferred DNA concentration of $AM$ with the median abundance of $AM$ across all training data. For example, if the marker features are A, B, C and D. wherein A is the anchor marker ($AM$) having a median abundance of $ABN_{AM}$, then the abundances of the marker features B, C and D will be computed as;

$$ABN_B = (Inferred\ conc.\ of B / Inferred\ conc.\ of A) \times ABN_{AM}$$

$$ABN_C = (Inferred\ conc.\ of C / Inferred\ conc.\ of A) \times ABN_{AM}$$

$$ABN_D = (Inferred\ conc.\ of D / Inferred\ conc.\ of A) \times ABN_{AM}$$

Further at step 214, a minimum number of multiplexed qPCR runs required for quantifying the relative abundance of each microbial taxonomic group belonging to the frugal set of markers are determined based on a unique number of microbial taxonomic groups constituting the frugal set of markers. The minimum number is determined using the formula:

$$1 + \lceil (n - 4)/4 \rceil$$

, where '$n$' is the unique number of microbial taxonomic groups constituting the frugal set of markers, and wherein each multiplexed qPCR run is configured to determine, in the test biological sample, the relative abundance of a predetermined subset of the microbial taxonomic groups constituting the frugal set of markers.

At step 216 of the method 200, a ranking of the microbial taxonomic groups constituting the frugal set of markers is determined. The ranking is utilized for determining the subset of microbial taxonomic groups from amongst the taxonomic groups constituting the frugal set of markers whose abundance is to be probed, in the test biological sample, using more than one multiplexed qPCR runs. The ranking is determined based on one or more of (i) a median abundance of each of the frugal set of markers from the train biological samples, and (ii) a frequency of occurrence of each of the frugal set of markers across the one or more classification sub-models constituting the ensemble classification model. More specifically, the one or more predetermined microbial marker sequences (from amongst the set of predetermined microbial marker sequences) that has/ have the highest (or relatively higher) median abundance or frequency of occurrence (as compared to the median abundance(s) or the frequency of occurrence of each microbial marker sequences in the remaining set of predetermined microbial marker sequences) across the plurality of training stool samples is/ are common to the multiplex qPCR runs.

It should be appreciated that the collection of the plurality of training biological sample, building the ensemble model, and subsequently identifying the frugal set of biomarkers, through steps 202 to 216, are the one-time process.

At step 218 of the method 200, the relative abundance of each of the microbial taxonomic groups constituting the frugal set of markers is determined in the test biological sample by performing the determined minimum number of multiplexed qPCR runs utilizing the designed set of qPCR probes, and ranking of the microbial taxonomic groups constituting the frugal set of markers is designed.

And finally at step 220 of the method 200, the test biological sample is classified to one of the first class or the second class utilizing the ensemble classification model, and the determined relative abundance of the each of the microbial taxonomic groups constituting the frugal set of markers in the test biological sample.

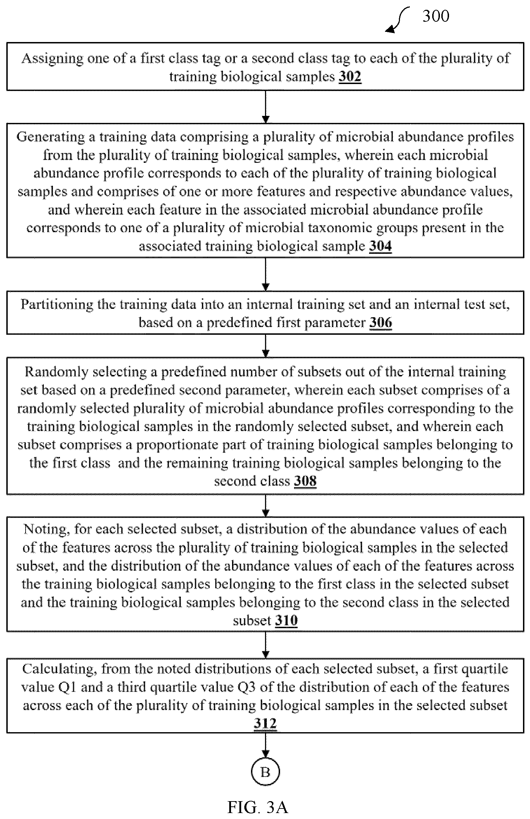

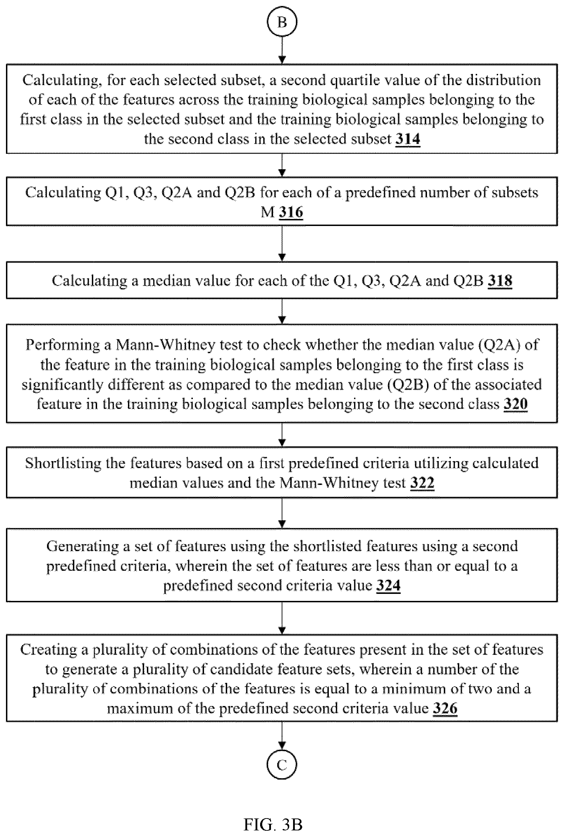

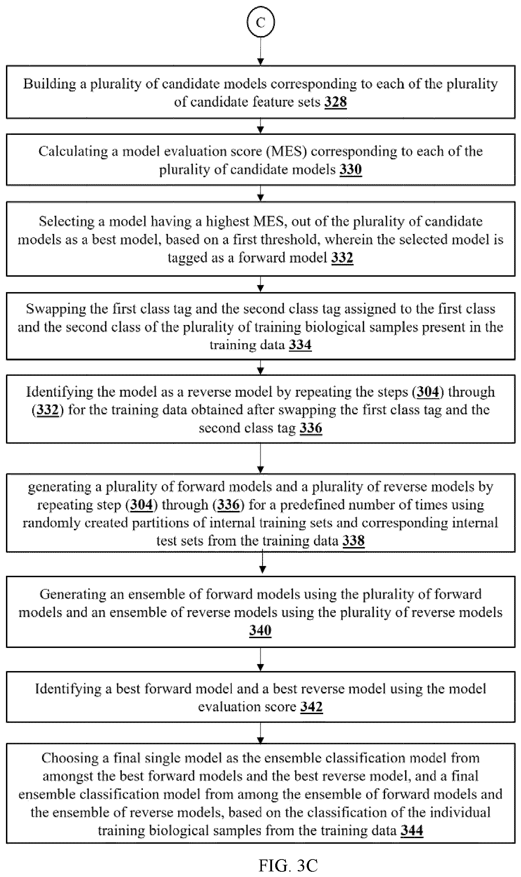

According to an embodiment of the disclosure, the ensemble classification model is built only one time. FIG. 3A-3C are flowcharts illustrating the steps involved in building an ensemble classification model, according to some embodiments of the present disclosure. The method for building the ensemble classification model accepts data in form of a feature table for multiple observations (or training biological samples) wherein each observation/training biological sample is defined by 'N' features (F) which are either or both of continuous and counted variables with ($N \geq 1$). In case of training data (TR), each of the training biological samples/observations further have a preassigned class/category/state which is binary in nature, i.e., the first class (A) (e.g., affiliating to healthy samples category) and the second class (B) (e.g., affiliating to unhealthy or disordered samples category). In case of test data (TS) or data received during actual deployment of the method, the model(s) built based on training data predicts the class/category/state of the training biological samples/observations. During training process, the following steps are followed:

Initially at step 302, a first class tag or a second class tag is assigned to each of the training biological sample in the plurality of training biological samples. At step 304, the training data comprising of a plurality of microbial abundance profiles corresponding to each of the plurality of training biological samples, is generated. Each microbial abundance profile is corresponding to each of the plurality of training biological sample and comprising of one or more features and respective abundance values of the features. Each feature in the microbial abundance profile corresponds to one of a plurality of microbial taxonomic groups present in the associated training biological sample.In the next step 306, the training data (TR) is randomly partitioned into two sets - namely, an internal-train (ITR) and an internal-test (ITS), based on a predefined first parameter '$L_1$'. Wherein, $L_1$% training biological samples from the total training data constitute the ITR set and (100 - $L_1$) % of the training biological samples constitute the ITS set. Furthermore, the random partitioning into ITR and ITS sets is performed using a stratified sampling approach with the intent of preserving the relative proportion of training biological samples belonging to the first class (A) or the second class (B) in the total training data in these newly drawn subsets.

In the next step 308, a predefined number of subsets are randomly selected out of the internal training set based on a pre-defined second parameter ($L_2$). Each of the subset comprises a randomly selected plurality of microbial abundance profiles corresponding to the plurality of training biological samples in the randomly selected subset, and wherein each of the subset comprises a proportionate part of training biological samples belonging to the first class (A) and the remaining training biological samples belonging to the second class (B). Thus, from ITR, 'M' randomly drawn subsets $ITRS_i$ (e.g., $ITRS_1$, $ITRS_2$, $ITRS_3$ .... $ITRS_M$), each containing S training biological samples are further generated, wherein $S = L_2$ % of the training biological samples present in ITR. For example, the values of $L_2$ and M are 80 % and 100 respectively and have also been used as default values during the validation experiments.

In the next step 310, for each selected subset, a distribution of the abundance values of each of the features across the plurality of training biological samples in the selected subset, and the distribution of the abundance values of each of the features across the training biological samples belonging to the first class (A) in the selected subset and the training biological samples belonging to the second class (B) in the selected subset are noted. Thus, from each subset $ITRS_i$ (where $i = 1, 2, 3, ..., M$), wherein there are total S training biological samples, each of which are described by N features ($F_j$) (where $j = 1, 2, 3, ..., N$),the distributions of each of the features ($ITRS_iDF_j$) across S training biological samples are noted. Similarly, from each subset $ITRS_i$, wherein there are $S_A$ training biological samples belonging to the first class (A) and $S_B$ training biological samples belonging to the second class (B), each of the training biological samples being described by $N$ features ($F_j$; $j$ = 1, 2, 3, ..., $N$), the distributions of each of the features ($ITRS_iD_AF_j$) across $S_A$ training biological samples, and the distributions of each of the features ($ITRS_iD_BF_j$) across $S_B$ training biological samples are noted.

In the next step 312, from the noted distributions of each selected subset, a first quartile value ($Q1$) and a third quartile value ($Q3$) of the distribution of each of the features is calculated across each of the plurality of training biological samples in the selected subset. In an example, the respective first quartile value ($Q1$) and the third quartile value ($Q3$) of $ITRS_iDF_j$ may also be referred as $Q1ITRS_iDF_j$ and $Q3ITRS_iDF_j$ respectively.

Furthermore, in the next step 314, for each selected subset, a second quartile value of the distribution of each of the features across the training biological samples belonging to the first class ($Q2_A$) in the selected subset and the training biological samples belonging to the second class ($Q2_B$) in the selected subset is calculated. Thus, in an example, the median value (in other words, the second quartile value) of ($ITRS_iD_AF_j$) is referred as $Q2ITRS_iD_AF_j$, and the median value of ($ITRS_iD_BF_j$) is referred as $Q2ITRS_iD_BF_j$.

In the next step 316, for the M subsets of $ITRS_j$, a total of $M$ values for each of $Q1ITRS_iDF_j$, $Q3ITRS_iDF_j$, $Q2ITRS_iD_AF_j$, and $Q2ITRS_iD_BF_j$, are calculated. Further at step 318, a median value ($\widetilde{Q1}_J$) is calculated for all calculated $Q1$, a median value ($\widetilde{Q3}_J$) is calculated for all calculated $Q3$, a median value ($\widetilde{Q2_{A_J}}$) is calculated for all calculated $Q2_A$ and a median value ($\widetilde{Q2_{B_J}}$) is calculated for all calculated $Q2_B$. Thus,

$$\widetilde{Q1}_j = \text{median of } \{Q1ITRS_1DF_j, Q1ITRS_2DF_j, Q1ITRS_3DF_j,... Q1ITRS_MDF_j\}$$

$$\widetilde{Q3}_j = \text{median of } \{Q3ITRS_1DF_j, Q3ITRS_2DF_j, Q3ITRS_3DF_j,... Q3ITRS_MDF_j\}$$

$$\widetilde{Q2_{A_J}} = \text{median of } \{Q2ITRS_1D_AF_j, Q2ITRS_2D_AF_j, Q2ITRS_3D_AF_j, .... Q2ITRS_MD_AF_j\}$$

$$\widetilde{Q2_{B_J}} = \text{median of } \{Q2ITRS_1D_BF_j, Q2ITRS_2D_BF_j, Q2ITRS_3D_BF_j, .... Q2ITRS_MD_BF_j\}$$

(where $i$ = 1,2,3,...,$M$; and $j$ = 1, 2, 3, ..., $N$)

In the next step 320, a Mann-Whitney test is performed to test if a median value of the feature ($F_j$) is significantly ($p<0.1$) different between the training biological samples belonging to the first class ($S_A$ ) and the training biological samples belonging to the second class ($S_B$ ) in each of the M randomly drawn subsets $ITRS_j$. Other statistical tests based on the nature of distribution (e.g., t-test for normal distribution), nature of sampling (e.g., Wilcoxon signed rank test for paired case and control training biological samples) or other methods of statistical comparison relevant for microbiome datasets (e.g., ALDEx2) can also be adopted.

In the next step 322, the features are shortlisted based on a first predefined criteria utilizing calculated median values and the Mann-Whitney test. The first predefined criteria comprises if a feature $F_j$ is observed to have significantly ($p<0.1$) different values in $S_A$ compared to $S_B$ in more than 70% of $M$ subsets, and if $\widetilde{Q2_{A_J}} >= Q2_{min}$ OR $\widetilde{Q2_{B_J}} >= Q2_{min}$ (using a pre-defined 'abundance' threshold and $Q2_{min}$ threshold (example value is 0 as described in the case study). $F_j$ is added to a set of shortlisted features ($SF$).

In the next step 324, a set of features is generated using the shortlisted features ($SF$) using a predefined second predefined criteria. An exemplary value of the predefined second predefined criteria is 15. Hence, in an embodiment, the set of features are less than or equal to 15. If the number of shortlisted features ($SF$) obtained in previous step satisfies the criteria $1 \leq SF \leq 15$, then the training process proceeds to model building with all the features in $SF$. If no shortlisted features ($SF$) are obtained in previous (i.e., $SF < 1$) then following step is performed with all the features $F_j$ for evaluating the ability of the features, when considered independently, to distinguish between training biological samples belonging to the first class (A) and the second class (B). Similarly, if the number of shortlisted features ($SF$) obtained in previous step exceeds fifteen ($SF > 15$) then following step is performed with all the shortlisted features ($SF$) for evaluating the ability of the features, when considered independently, to distinguish between training biological samples belonging to the first class (A) and the second class (B).

Steps for shortlisting the features in case of $SF < 1$ or $SF > 15$: For each of the features (obtained previously) taken individually, different threshold values are used to classify the training biological samples belonging to the set $ITR$, and the results are cumulated to construct a receiver operating characteristic curve (ROC curve) for each of the features. The area under the curve (AUC) of the ROC curve of any feature ($AUC^F$) is indicative of the utility of the feature to distinguish between training biological samples belonging to the first class (A) and the second class (B), and the same is computed for every feature. The shortlisted features (SF) set is modified to include only the top fifteen features from a list of features arranged in a descending order of the $AUC^F$ values.

In the next step 326, a plurality of combinations of the features present in the set of features is created to generate corresponding plurality of candidate feature sets (CF), wherein the plurality of combinations of features comprises a minimum of one and a maximum of 15 features. By definition the maximum possible candidate feature sets that can be created in this process is $K = 2^{15} - 1 = 32767$ (i.e., maximum value of K = 32767).

In the next step 328, a plurality of candidate models is built corresponding to each of the plurality of candidate feature sets. At step 330, a model evaluation score (MES) is calculated corresponding to each of the plurality of candidate models. For each candidate feature set $CF_K$, a corresponding candidate model $CM_K$ is built and evaluated as mentioned in the steps mentioned below.

Steps for evaluating the candidate model:

- Step 1: The values of the features $F_j$ constituting a candidate feature set defining the training biological samples in *ITR* are transformed to $F_j'$ such that - $\widetilde{Q1}_J$, $\widetilde{Q3}_J$, $\widetilde{Q2_{A_J}}$ and $\widetilde{Q2_{B_J}}$

$$F_j' = 0 \dots\dots\dots\dots\dots \text{ if } F_j < \widetilde{Q1}_J$$

$$F_j' = 1 \dots\dots\dots\dots\dots \text{ if } F_j > \widetilde{Q3}_J$$

$$F_j' = 0.5 \dots\dots\dots\dots\dots \text{ if } \widetilde{Q1}_J = \widetilde{Q3}_J$$

$$F_j' = \frac{F_j - \widetilde{Q1}_J}{\widetilde{Q3}_J - \widetilde{Q1}_J} \dots\dots\dots\dots\dots \text{ if } \widetilde{Q1}_J < F_j < \widetilde{Q3}_J$$

- Step 2: If for a feature $F_j$, it is observed that $\widetilde{Q2_{B_J}} > \widetilde{Q2_{A_J}}$, then the feature $F_j$ is tagged as a 'numerator' feature and added to a set of numerator features $F_{numerator}$. Else, feature $F_j$ is tagged as a 'denominator' feature and added to a set of denominator features $F_{denominator}$.
- Step 3: Each candidate model ($CM_K$) is constituted as a simple ratio function given below -

$$CM_K = \frac{\sum F_{numerator}}{\sum F_{denominator}} \text{ ... when } F_{numerator} > 0 \text{ and } F_{denominator} > 0 \text{ or,}$$

$$CM_K = \frac{\sum F_{numerator} + 1}{\sum F_{denominator} + 1} \text{ ... when either } F_{numerator} \text{ or } F_{denominator} = 0$$

wherein, $\sum F_{numerator}$ represents the sum of values of all numerator features for a particular training biological sample, and,

wherein, $\sum F_{denominator}$ represents the sum of values of all denominator features for a particular training biological sample.

For each of the features, a transformed value $F_j'$ as obtained above is used in the candidate model equation.

- Step 4: A candidate model c is used to generate candidate model scores ($CMS_K$) for each of the training biological samples in the set *ITR*. From the set of scores $CMS_K$, the top 10 percentile and bottom 10 percentile scores are removed as outliers and thereafter the maximum and minimum scores from the set $CMS_K$ are noted as $CMS_{K_{max}}$ and $CMS_{K_{min}}$ respectively.
- Step 5: Considering each of the scores in the set $CMS_K$ as a threshold (T), the model $CM_K$ is used to (re)classify the training biological samples in the training set (*ITR.*) such that -
- a training biological sample is classified into the first-class if *CMS >= T*
- or a training biological sample is classified into the second-class if *CMS < T* and based on a comparison of these classifications and the true/original classes of the training biological samples, Mathew's correlation coefficients (*MCC*) for each of the thresholds are calculated, to evaluate how well each of the thresholds can distinguish between training biological samples between the first class (A) and the second class (B).

- Step 6: The threshold ($T_{max}$) which provides the maximum absolute *MCC* value ($|MCC_{max}|$) is noted. If $|MCC_{max}| < 0.4$ for a candidate model $CM_K$, then the candidate model is discarded from further evaluation. Else, the $|MCC_{max}|$ value is considered as the 'train-*MCC*' value ($|MCC_{train}|$) for the model *ITS* and the model and its corresponding $T_{max}$ threshold is used to classify the training biological samples in the internal-test set (*ITS*). In another implementation of the process, the $MCC_{max}$ threshold may not be applied for retaining the candidate model for subsequent evaluation. Before classifying the training biological samples in the *ITS* set, the values of features characterizing the training biological samples of the *ITS* set are transformed using the method mentioned in step 17 while using the earlier obtained values of $\widetilde{Q1}_J$, $\widetilde{Q3}_J$, $\widetilde{Q2_{A_J}}$ and $\widetilde{Q2_{B_J}}$ from the *ITR* set.

- Step 7: The classification results on the training biological samples from the *ITS* set are compared against the true/original classes of the training biological samples (with pre-assigned labels), and the *MCC* for the model $CM_K$ and its corresponding $T_{max}$ threshold on the *ITS* training biological samples is calculated ($MCC_{test}$).

- Step 8: A model evaluation score *(MES)* for candidate model $CM_K$ is calculated as

$$MES = |(MCC_{train} + MCC_{test})| - |(MCC_{train} - MCC_{test})|$$

In the next step 332, the model $CM_K$ is tagged as a "strong model" if all the features in the corresponding candidate feature set satisfies the Mann-Whitney test based shortlisting criteria described above. Otherwise, if any of the features in the corresponding feature set fails to satisfy the Mann-Whitney test, the model $CM_K$ is tagged as a "weak model".

Further, the above process is repeated for candidate models and respective *MES* scores are used to rank all the models. The best model is subsequently chosen based on the *MES* score. In case, there are more than one model with the best *MES* score, the best model is chosen based on the following criteria (in order of preference):

    (a) the model with fewer number of features (i.e., based on a smaller candidate feature set) is chosen.
    (b) the model with lower $T_{max}$ (threshold value) is chosen.

Further, the best model obtained through above steps is tagged as a forward model ($MD_{fwd}$). The model $MD_{fwd}$ additionally constitutes its corresponding $T_{max}$ threshold, the $CMS_{Kmax}$ and $CMS_{Kmin}$ values, and the $\widetilde{Q1}_J$, $\widetilde{Q3}_J$, $\widetilde{Q2_{A_J}}$ and $\widetilde{Q2_{B_J}}$ values corresponding to the *ITR* set.

In the next step 334, the tags assigned to the first class (A) and the second class (B) of the plurality of training biological samples present in the training data are swapped. At step 336, the steps 304 through 332 are repeated to determine the best model are repeated after swapping the class labels (A <-> B) for the entire training set (*TR*) to obtain a best model tagged as the reverse model ($MD_{rev}$). The model $MD_{rev}$ additionally constitutes its corresponding $T_{max}$ threshold, the $CMS_{Kmax}$ and $CMS_{Kmin}$ values, and the $\widetilde{Q1}_J$, $\widetilde{Q3}_J$, $\widetilde{Q2_{A_J}}$ and $\widetilde{Q2_{B_J}}$ values corresponding to the *ITR* set.

At step 338, a plurality of forward models and a plurality of reverse models are generated, by repeating step (304) through (336) for a predefined number of times using randomly created partitions of internal training sets and corresponding internal test sets from the training. The steps (304) through (336) are iterated '*R*' times using multiple randomly partitioned *ITR* and *ITS* sets generated initially. After each iteration, (i) the features constituting the models $MD_{fwd}$ and the models $MD_{rev}$ obtained in the current iteration (*r*) are compared against, and if necessary, appended to, a set of unique features $F_{unq}$ that consists of respective features constituting the $MD_{fwd}$ and $MD_{rev}$ obtained in earlier iterations (i.e., up to iteration *r* - 1). After '*R*' iterations, a plurality of forward models and a plurality of reverse models are generated for a predefined number of times using randomly partitioned internal training set and the internal test set. The iterations proceed while the value of R satisfies the following criteria -

    (i)

$$R \leq R_{max}$$

    (ii) ($|F_{unq}|$ after iteration R) > ($|F_{unq}|$ after iteration $R - R_{unq}$)
    (iii) $|F_{unq}|$ after iteration no. $R <= Fet_{max}$

Wherein, $R_{max}$ is a pre-defined parameter indicating the maximum number of iterations allowed; $R_{unq}$ is a pre-defined parameter indicating the maximum number of iterations allowed without any cumulative increase in the number of unique features $|F_{unq}|$ in the models being generated in consecutive iterations; and $Fet_{max}$ is a pre-defined parameter indicating the maximum allowed value of $|F_{unq}|$ (i.e., the no. of unique features cumulated through the iterative process).

In the next step 340, an ensemble of forward models is generated using the plurality of forward models and an ensemble of reverse models is generated using the plurality of reverse models. This is referred as an ensemble of forward models (ENS-$MD_{fwd}$) and an ensemble of reverse models (ENS-$MD_{rev}$).

At step 342, the best models from each of these ensembles, i.e., the best of the forward models ($BMD_{fwd}$) and the best of the reverse models ($BMD_{rev}$) respectively, are identified.

If all models in an ensemble are "weak models", the best model from the ensemble ($BMD$) is chosen by ranking the models based on their model evaluation scores and associated criteria. Also, if an ensemble contains more than one "strong models", then only those strong models are considered for ranking based on their model evaluation scores and associated criteria as mentioned above, and the best model from the ensemble ($BMD$) is thereby chosen. The associated criteria include: (i) the model with fewer number of features (i.e., based on a smaller candidate feature set) is chosen, (ii) the model with lower $T_{max}$ (threshold value) is chosen.

In the next step 344, a final single model ($FM_{single}$) is chosen as the ensemble classification model from amongst the best forward model and the best reverse model based on how they classify the individual training biological samples from the training data ($TR$). Once the best models from each of the ensemble of forward models and the ensemble of reverse models, i.e., the best of the forward models ($BMD_{fwd}$) and the best of the reverse models ($BMD_{rev}$) are identified, the final single model ($FM_{single}$) is chosen from amongst $BMD_{fwd}$ and $BMD_{rev}$ based on how well they can classify the individual training biological samples from the entire training set ($TR$). The AUC value for ROC curves for each of these two models are computed based on the predicted model scores for the training set (TR) training biological samples and their pre-assigned classes. The model having the best AUC for ROC value is selected as the final single model ($FM_{single}$). If both $BMD_{fwd}$ and $BMD_{rev}$ have the same AUC value, $BMD_{fwd}$ is chosen as $FM_{single}$.

In an alternate implementation $FM_{single}$ can be chosen based whether $BMD_{fwd}$ or $BMD_{rev}$ obtains a higher $MCC$ value while classifying the $TR$ training biological samples. Once the $FM_{single}$ model has been chosen, for classification of any test biological samples from a test set $(TS)$ or any sample data received during actual deployment, the $FM_{single}$ model is used after:

(a) appropriately transforming the features corresponding to the training biological sample being classified using the $\widetilde{Q1}_J$, $\widetilde{Q3}_J$, $\widetilde{Q2_{A_J}}$ and $\widetilde{Q2_{B_J}}$ values corresponding to the $FM_{single}$ model,

(b) limiting the model score between a maximum of $CMS_{Kmax}$ and a minimum of $CMS_{Kmin}$ values corresponding to the $FM_{single}$ model, and

(c) classification based on the model score using its corresponding threshold $T_{max}$.

According to an embodiment of the disclosure, the ensemble of forward models (ENS-$MD_{fwd}$) and the ensemble of reverse models (ENS-$MD_{rev}$) are also evaluated for corresponding collective classification efficiencies using an ensemble model scoring. In the ensemble scoring method, each of the models ($MD$) constituting an ensemble (ENS) are used to generate a model score ($MS$) for each of the training biological samples from the entire $TR$ set. For any specific training biological sample the values of the features corresponding to the training biological sample are appropriately transformed using the $\widetilde{Q1}_J$, $\widetilde{Q3}_J$, $\widetilde{Q2_{A_J}}$ and $\widetilde{Q2_{B_J}}$ values corresponding to the model MD. The model scores (MS) are then transformed into scaled model scores $(SMS)$ having values between -1 and +1, using the following procedure:

$$SMS = (MS - T_{max})/( CMS_{K_{max}} - T_{max}), \ldots\ldots \text{ when } MS >= T_{max}, \text{ and}$$

$$SMS = (MS - T_{max})/( T_{max} - CMS_{K_{min}}), \ldots\ldots \text{ when } MS < T_{max},$$

Wherein, $T_{max}$, $CMS_{K_{max}}$, and $CMS_{K_{min}}$ values corresponding to the respective model is used.

Let $SMS_{avg}$ be the average of all $SMS$ obtained using all models in ENS for a particular training biological sample.

When using Forward model [ENS - $MD_{fwd}$],

$$SMS_{avg} = SMS_{avg} * (+1)$$

If $SMS_{avg} >= 0$, training biological sample is classified as 'B'

If $SMS_{avg} < 0$, training biological sample is classified as 'A'

When using Reverse model [ENS - $MD_{rev}$]:

$SMS_{avg} = SMS_{avg} * (-1)$ If $SMS_{avg} > 0$, training biological sample is classified as 'B' If $SMS_{avg} <= 0$, training biological sample is classified as 'A'

If all models in one of the ensembles are weak models, then the other one having (one or more) strong models is selected as a final ensemble model ($FM_{ens}$), and subsequently used for classification of any test biological samples from the test set *(TS)* or any sample data received during actual deployment of the method, using the scoring and classification process mentioned in above paragraph. If both ensembles have constituent strong models, then both the ensembles are evaluated for their efficiency by scoring them on all individual training biological samples in *TR.* The AUC value for ROC curves for each of these two ensembles are computed based on the predicted $SMS_{avg}$ for all the training set (*TR*) training biological samples and their pre-assigned classes. The ensemble of models having the best AUC for ROC value is selected as the final ensemble model ($FM_{ens}$). In case both ENS-$MD_{fwd}$ and ENS-$MD_{rev}$ exhibit equal AUC values then ENS-$MD_{fwd}$ is chosen as the final ensemble model ($FM_{ens}$). In an alternate implementation, $FM_{ens}$ can be chosen based whether ENS-$MD_{fwd}$ and ENS-$MD_{rev}$ obtains a higher average MCC value for their respective constituent models while classifying the *TR* training biological samples.

Thus, either the $FM_{single}$ model or $FM_{ens}$ ensemble of models can be used for classification of any test biological samples from a test set *(TS)* or any sample data received during actual deployment.

According to an embodiment of the disclosure, the system 100 comprises the recommendation module 114. The recommendation module 114 generates a feature set that can potentially be targeted/ modulated for therapeutic purposes, based on the diagnosed feature profile(s). The recommendation module 114 is configured to design/recommend a pre-biotic or pro-biotic formulation which can modulate the taxonomic abundances of a microbiome in a diseased individual towards a control-like distribution.

According to an embodiment of the disclosure, the method for designing/recommending a recommendation with a personalized therapeutic formulation to the individual (in instances where the ML model classifies a test sample to a 'disease' class) is explained as follows:

At step 1, pair-wise correlations (using the Pearson's and/or spearman's correlation index) are computed between abundances of features (i.e., organisms/ taxa/ microbes) constituting the ML model and the abundances corresponding to the complete set of microbes computed individually from (a) the subset of biological samples corresponding to the healthy class i.e. the class of samples that were taken from individuals not having disease, and (b) the diseased class i.e. the class of samples that were taken from individuals having disease). Wherein the samples belonging to the healthy and diseased classes are used as training data for generating the ML model.

At step 2, positive and negative interactions between features (i.e., organisms/ taxa/ microbes) constituting the ML model and all other taxa in the healthy and the diseased class of training samples (individually) are deduced using critical correlation (r) value as the cut-off (as taught in Batushansky et al., 2016), such that inter-taxa correlation index values greater than +*r* value are affiliated as 'positive interactions', while those less than -*r* value are affiliated as 'negative interactions'.

At step 3, the steps 1 and 2 are repeated 1000 times and only those interactions are considered relevant that appear in at least 70% of iterations with a BH (Benjamini-Hochberg) corrected p-value cut-off of 0.1 are retained (hereafter referred to as model taxa interactions corresponding to health and diseased class of samples).

At step 4, thereafter, following set of rules (indicated in Table 1 below) are used to arrive at the relevant candidate using the retained model taxa interactions:

Table 1

| Probiotic Candidates | Antibiotic Candidates |
|---|---|
| $(M_H - C_T)_{HP}$ && $(M_H - C_T)_{DP}$ <br> $(M_H - C_T)_{DP}$ | $(M_H - C_A)_{HN} \| (M_H - C_A)_{DN}$ <br> $(M_D - C_A)_{HP} \| (M_D - C_A)_{DP}$ |

(continued)

| Probiotic Candidates | Antibiotic Candidates |
|---|---|
| $(M_D - C_T)_{HN}$ && $(M_D - C_T)_{DN}$ | |

From Table 1,

$M_H$ represents a model taxon having significantly higher abundance in healthy class;

$M_D$ represents a model taxon having significantly higher abundance in diseased (unhealthy) class;

$C_T$ represents a potential therapeutic candidate;

$C_A$ represents a potential antibiotic target candidate;

$M_H$ - $C_T$ represents an interaction between a model taxon (abundant in healthy class) with a potential therapeutic candidate;

$M_D$ - $C_T$ represents an interaction between a model taxon (abundant in diseased class) with a potential therapeutic candidate;

$M_D$ - $C_A$ represents an interaction between a model taxon (abundant in diseased class) with a potential antibiotic target candidate;

$M_H$ - $C_A$ represents an interaction between a model taxon (abundant in healthy class) with a potential antibiotic target candidate;

$HP$ represents a positive interaction in a healthy environment population;

$HN$ represents a negative interaction in a healthy environment population;

$DP$ represents a positive interaction in a diseased environment population; and

$DN$ represents a negative interaction in a diseased environment population.

According to an embodiment of the present disclosure, the method 200 can also be explained with the help of experimental results. The method for identification of a frugal set of combination of biomarkers has been tested with clinical datasets pertaining to two diseases, i.e., breast cancer and PCOS. The results of these two datasets have been provided below.

Case study 1: Raw sequenced reads were taken from the publicly available breast cancer study. The raw sequenced data were first quality filtered using Prinseq-lite tool and taxonomic affiliations of the reads in the sample were then determined using Ribosomal Database Project version 2.12. Finally, the abundance table was generated for estimating the abundance of each taxonomic feature in every sample. The raw abundance values were then rarefied at a minimum sampling depth/ rarefaction depth (i.e., minimum library size determined after the sequencing run) using 'qiime feature-table rarefy' function in qiime tools of qiime2 package. This was done in order to remove any bias in the dataset and rarefied values were then further used for model generation.

Predicting breast cancer from the above-mentioned data (Stool microbiome; Total number of samples = 94; Cases = 47; Controls = 47). The classification models were built using both the methodology described in the present disclosure and Random Forest (RF) methodology for comparison. Table 1 shows evaluation results for 100 Models built with Train-Test sets which were partitioned in 90:10 proportion by stratified random selection of samples, where AUC depicts Area Under Curve and MCC depicts Matthew's correlation coefficient.

| Breast Cancer | Mean Feature Count in the model | Mean AUC | Std. Dev AUC | Mean MCC | Std. Dev MCC |
|---|---|---|---|---|---|
| RF (all features) | 201 | 0.72 | 0.16 | 0.31 | 0.31 |
| RF (15 features) | 15 | 0.71 | 0.14 | 0.31 | 0.31 |
| ENRICH (single) | 4 | 0.73 | 0.15 | 0.36 | 0.25 |
| ENRICH (ensemble) | 16 | 0.73 | 0.14 | 0.35 | 0.25 |

Table 1 shows evaluation results for 100 Models built with Train-Test sets which were partitioned in 90:10 proportion by stratified random selection of samples

Case study 2: Raw sequenced reads were taken from the publicly available PCOS study. The raw sequenced data were first quality filtered using Prinseq-lite tool and taxonomic affiliations of the reads in the sample were then determined using Ribosomal Database Project version 2.13. Finally, the abundance table was generated for estimating the abundance of each taxonomic feature in every sample. The raw abundance values were then percent normalized to remove any bias in the dataset and were then further used for model generation.

Predicting PCOS from the above-mentioned data (Salivary microbiome; Total number of samples = 43; Cases = 24; Controls = 19). The classification models were built using both the methodology described in the present disclosure and Random Forest for comparison. The table 2 shows evaluation results for 100 Models built with Train-Test sets which were partitioned in 90:10 proportion by stratified random selection of samples.

| PCOS | Mean Feature Count | Mean AUC | Std. Dev AUC | Mean MCC | Std. Dev MCC |
|---|---|---|---|---|---|
| RF (all features) | 194 | 0.74 | 0.22 | 0.36 | 0.41 |
| RF (15 features) | 15 | 0.74 | 0.21 | 0.42 | 0.28 |
| ENRICH (single) | 3 | 0.75 | 0.17 | 0.41 | 0.26 |
| ENRICH (ensemble) | 8 | 0.78 | 0.18 | 0.42 | 0.28 |

The table 2 shows evaluation results for 100 Models built with Train-Test sets which were partitioned in 90:10 proportion by stratified random selection of samples.

According to an embodiment of the disclosure, the system 100 can also be explained with the help of experimental workflow for a woman for breast cancer risk screening.

Step 1: Obtain a stool sample from an individual for whom breast cancer risk screening is intended.

Step 2: Quantify the raw abundances of various microbial taxonomic groups in the stool sample. Methodology for this involves extraction of microbial DNA contents from the collected stool sample followed by amplification and sequencing of either full-length or specific variable regions of the bacterial 16S rRNA marker genes using a next-generation sequencing platform or by using the multiplex qPCR-based quantification methodology. In either case, the sequencing depth (i.e., number of reads obtained by sequencing the microbial DNA content of the stool sample) obtained should exceed a pre-defined threshold, wherein the said threshold refers to the rarefaction depth that needs to be determined and/ or applied to adjust for differences in library sizes across samples in current the sequencing run or for removing the sequencing bias).

TABLE 3: Raw abundance of features in a test sample

| Features | Raw Abundance |
|---|---|
| Butyricicoccus | 159 |
| Clostridium_IV | 95 |
| Clostridium_sensu_stricto | 0 |
| Faecalibacterium | 5936 |
| Flavonifractor | 40 |
| Gemmiger | 465 |
| Lachnospiracea_incertae_sedis | 899 |
| Murimonas | 1 |
| Oscillibacter | 148 |
| .. | |
| .. | |
| Ruminococcus | 329 |
| Sporobacter | 0 |

Step 3: Rarefy the abundances of various taxa employing rarefaction depth i.e., minimum library size determined after the sequencing run wherein, rarefaction was done using 'qiime feature-table rarefy' function in qiime tools of qiime2 package.

TABLE 4: Rarefied abundance of features in the test sample

| Features | Rarefied Abundance |
|---|---|
| Butyricicoccus | 50 |
| Clostridium_IV | 34 |

(continued)

| Features | Rarefied Abundance |
|---|---|
| Clostridium_sensu_stricto | 0 |
| Faecalibacterium | 2221 |
| Flavonifractor | 10 |
| Gemmiger | 165 |
| Lachnospiracea_incertae_sedis | 306 |
| Murimonas | 0 |
| Oscillibacter | 46 |
| .. | |
| .. | |
| Ruminococcus | 131 |
| Sporobacter | 0 |

Step 4: From the rarefied abundance table 4, retain abundances of only the subset of taxa which overlap with the list of three taxa that are provided against 'Single Best Training Model' as mentioned below in Table 5.

TABLE 5: Model characteristics of features in the Single Best Training Model

| Features | Murimonas | Clostridium_sensu_stricto | Lachnospiracea_incertae_ sedis |
|---|---|---|---|
| $Q1$ | 0 | 1 | 209.5 |
| $Q3$ | 1 | 5 | 436.5 |
| $Q2_A$ | 0 | 0 | 336 |
| $Q2_B$ | 1 | 3 | 252 |
| Numerator/ Denominator Feature | Numerator | Numerator | Denominator |
| Min Model Score | 0.5 | | |
| Max Model Score | 5.321305 | | |
| Threshold | 1.402786 | | |
| Model Type | Reverse | | |

As an example, assume that the three taxa in the taxonomic abundance profile obtained by processing the stool sample (in the manner mentioned in Steps 1 and 2) had the following rarefied abundances:

Abundance of Murimonas (i.e., feature 1 in training model) in collected stool sample: 0.000000
Abundance of Clostridium_sensu_stricto (i.e., feature 2 in training model) in collected stool sample: 0.000000
Abundance of Lachnospiracea_ incertae_sedis (i.e., feature 3 in training model) in collected stool sample: 306.000000

Step 5: Using $Q1$ and $Q3$ values corresponding to each training model feature in the single best model (as mentioned in Table 5), and applying the transformation as mentioned above to the rarefied abundances, results in the following:

Transformed abundance (FMurimonas): 0.000000
Transformed abundance (FClostridium_sensu_stricto): 0.000000
Transformed abundance (FLachnospiracea_incertae_sedis): 0.425110

The transformed abundance of individual features as obtained above are then used appropriately in the candidate model equation ($CM_K$) (as replicated below), and numerator and denominator sums are computed. In this case, the values obtained are as follows: Since Numerator sum = 0 and Denominator sum = 0.425110 in this case, a value of 1 is added to both numerator and denominator.

$$CM_K = \frac{\sum F_{numerator}}{\sum F_{denominator}} \quad \text{... when } F_{numerator} > 0 \text{ and } F_{denominator} > 0 \text{ or,}$$

$$CM_K = \frac{\sum F_{numerator}+1}{\sum F_{denominator}+1} \quad \text{... when either } F_{numerator} \text{ or } F_{denominator} = 0$$

Numerator sum: 1.000000
Denominator sum: 1.425110

Step 6: The sample model score (*MS*) is computed next using above Numerator sum and Denominator sum. The sample model score (*MS*) is then transformed into scaled model score (*SMS*) (having values between -1 and +1, using following rules:

$$SMS = (MS - T_{max})/(CMS_{K_{max}} - T_{max}), \ldots \text{ when } MS >= T_{max},$$

and

$$SMS = (MS - T_{max})/(T_{max} - CMS_{K_{min}}), \ldots \text{ when } MS < T_{max},$$

Wherein, $T_{max}$, $CMS_{K_{max}}$, and $CMS_{K_{min}}$ values corresponding to the respective model is used.
For this purpose, the values of threshold: 1.402786,
Maximum model score: 5.321305, and Minimum model score: 0.500000 for single best model are employed.
Model score (*MS*): 0.701700
Scaled model score (*SMS*): -0.776580

Step 7: The *SMS* is then used for predicting the category of risk for the individual from whom the stool sample was obtained i.e., healthy or 'at-risk for breast cancer'. Since both forward model and reverse model are evaluated. Here in this case, final selected single best model is a reverse model, hence the final prediction score value is calculated as (*SMS* *-1)

Final pred_score is 0.776580.
Since the value is >0, the prediction class is "B" i.e., Risk Category is "Breast Cancer"

Following the same series of steps, if the value of *SMS* is less than 0 then the prediction class will be "A" and the category of risk for the individual from whom the stool sample was obtained will be "Healthy".

Step 8: Similarly, for Ensemble model, all the steps are repeated for all the single models in the ensemble and finally mean of all the Final prediction score calculated using sample model scores (*SMS*) and the class prediction is done based on final mean prediction score obtained.

The embodiments of present disclosure herein address unresolved problem of lengthy and time taking process of designing the artificially structured materials. The embodiment thus provides a method and system for designing artificially structured materials with customized functionalities. The method is faster and computationally less expensive than standard metamaterial simulations used in the prior art.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

## Claims

1. A method (200) for identifying and utilizing a frugal set of markers for classification of a test biological sample, the method comprising:

collecting a plurality of train biological samples comprising of a first subset of samples corresponding to a first class and a second subset of samples corresponding to a second class (202), wherein the first class belongs to diseased or anomaly cases and the second class belongs to healthy controls or vice versa;
extracting a microbial Deoxyribonucleic Acid (DNA) from each of the plurality of train biological samples (204);
sequencing the microbial DNA associated with each of the plurality of train biological samples, using a sequencer, to obtain a DNA sequence data corresponding to each of the plurality of train biological samples (206);
analyzing, via one or more hardware processors, the DNA sequence data corresponding to each of the plurality of train biological samples, to generate a plurality of microbial abundance profiles from the plurality of train biological samples, wherein each of the plurality of microbial abundance profiles corresponds to each of the plurality of train biological samples and comprises of abundance values associated with a plurality of individual microbial taxonomic groups corresponding to each of the plurality of train biological samples (208);
building, via the one or more hardware processors, an ensemble classification model by training a machine learning (ML) model using the plurality of microbial abundance profiles associated with the plurality of train biological samples, wherein the ensemble classification model comprises of a set of no more than a predefined number of microbial taxonomic groups as the frugal set of markers, wherein the predefined number of microbial taxonomic groups is a subset of the plurality of individual microbial taxonomic groups, wherein the predefined number of microbial taxonomic groups is the subset of markers not exceeding 15, and wherein the ensemble classification model comprises of one or more classification sub-models and wherein the ensemble classification model is configured to classify the test biological sample to one of the first class and the second class (210), and wherein the ensemble classification model is built by:

(i) assigning a first class tag or a second class tag to each of the plurality of training biological samples (302);
(ii) generating a training data comprising a plurality of microbial abundance profiles from the plurality of training biological samples, wherein each microbial abundance profile corresponds to each of the plurality of training biological samples and comprises of one or more features and respective abundance values, and wherein each feature in the associated microbial abundance profile corresponds to one of a plurality of microbial taxonomic groups present in the associated training biological sample (304);
(iii) partitioning the training data into an internal training set and an internal test set, based on a predefined first parameter (306);

(iv) randomly selecting a predefined number of subsets out of the internal training set based on a predefined second parameter, wherein each subset comprises of a randomly selected plurality of microbial abundance profiles corresponding to the training biological samples in the randomly selected subset, and wherein each subset comprises of a proportionate part of training biological samples belonging to the first class and the remaining training biological samples belonging to the second class (308);

(v) noting, for each selected subset, a distribution of the abundance values of each of the features across the plurality of training biological samples in the selected subset, and the distribution of the abundance values of each of the features across the training biological samples belonging to the first class in the selected subset and the training biological samples belonging to the second class in the selected subset (310);

(vi) calculating, from the noted distributions of each selected subset, a first quartile value $Q1$ and a third quartile value $Q3$ of the distribution of each of the features across each of the training biological samples in the selected subset (312);

(vii) calculating, for each selected subset, a second quartile value of the distribution of each of the features across the training biological samples belonging to the first class $Q2_A$ in the selected subset and the training biological samples belonging to the second class $Q2_B$ in the selected subset (314);

(viii) calculating $Q1$, $Q3$, $Q2_A$ and $Q2_B$ for each of a predefined number of subsets $M$ (316);

(ix) calculating a median value for each of the $Q1$, $Q3$, $Q2_A$ and $Q2_B$ (318);

(x) performing a Mann-Whitney test to check whether the median value ($Q2_A$) of the feature in the training biological samples belonging to the first class is significantly different as compared to the median value ($Q2_B$) of the associated feature in the training biological samples belonging to the second class (320);

(xi) shortlisting the features based on a first predefined criteria utilizing calculated median values and the Mann-Whitney test (322);

(xii) generating a set of features using the shortlisted features using a second predefined criteria, wherein the set of features are less than or equal to a predefined second criteria value (324);

(xiii) creating a plurality of combinations of the features present in the set of features to generate a plurality of candidate feature sets, wherein a number of the plurality of combinations of the features is equal to a minimum of two and a maximum of the predefined second criteria value (326);

(xiv) building a plurality of candidate models ($CM_K$) corresponding to each of the plurality of candidate feature sets (328);

(xv) calculating a model evaluation score *(MES)* corresponding to each of the plurality of candidate models (330);

(xvi) selecting a model having a highest *MES,* out of the plurality of candidate models as a best model, based on a first threshold ($T_{max}$), wherein the selected model is tagged as a forward model (332);

(xvii) swapping the first class tag and the second class tag assigned to the first class and the second class of the plurality of training biological samples present in the training data (334);

(xviii) identifying and subsequently tagging the model as a reverse model by repeating the steps (ii) through (xvi) for the training data obtained after swapping the first class tag and the second class tag (336);

(xix) generating a plurality of forward models and a plurality of reverse models by repeating step (ii) through (xviii) for a predefined number of times using randomly created partitions of internal training sets and corresponding internal test sets from the training data (338);

(xx) generating an ensemble of forward models (ENS-$MD_{fwd}$) using the plurality of forward models and an ensemble of reverse models (ENS-$MD_{rev}$) using the plurality of reverse models (340);

(xxi) identifying a best forward model and a best reverse model using the model evaluation score (342); and

(xxii) choosing a final single model ($FM_{single}$) from amongst the best forward models and the best reverse model, and a final ensemble classification model ($FM_{ens}$) from among the ensemble of forward models and the ensemble of reverse models, based on the classification of the individual training biological samples from the training data (344);

designing, via the one or more hardware processors, a set of quantitative polymerase chain reaction (qPCR)-probes corresponding to each microbial taxonomic group constituting to the frugal set of markers (212);

determining, via the one or more hardware processors, a minimum number of multiplexed qPCR runs required for quantifying a relative abundance of each microbial taxonomic group belonging to the frugal set of markers, based on a unique number of microbial taxonomic groups constituting the frugal set of markers, wherein each multiplexed qPCR run is configured to determine the relative abundance of the predetermined subset of the microbial taxonomic groups constituting the frugal set of markers, in the test biological sample (214);

determining, via the one or more hardware processors, a ranking of each of the microbial taxonomic groups constituting the frugal set of markers, based on one or more of (i) a median abundance of each of the frugal set of markers from the plurality of train biological samples, and (ii) a frequency of occurrence of each of the frugal set of

EP 4 451 285 B1

markers across the one or more classification sub-models constituting the ensemble classification model, wherein the ranking is utilized for determining the subset of microbial taxonomic groups from amongst the taxonomic groups constituting the frugal set of markers whose abundance is to be probed, in the test biological sample, using more than one multiplexed qPCR runs (216);

determining, via the one or more hardware processors, the relative abundance of each of the microbial taxonomic groups constituting the frugal set of markers in the test biological sample, by performing the determined minimum number of multiplexed qPCR runs utilizing a designed set of qPCR probes, and the ranking of each of the microbial taxonomic groups constituting the frugal set of markers (218); and

classifying, via the one or more hardware processors, the test biological sample to one of the first class and the second class, utilizing the ensemble classification model, based on the relative abundance of each of the microbial taxonomic groups constituting the frugal set of markers in the test biological sample (220).

2. The method (200) as claimed in claim 1, wherein the minimum number of multiplexed qPCR runs is determined using a formula: $1 + \lceil (n-4)/4 \rceil$, where n is the unique number of microbial taxonomic groups constituting the frugal set of markers.

3. The method (200) as claimed in claim 1, further comprises:

classifying each of the set of shortlisted features using a second threshold value different from the first threshold ($T_{max}$); and

cumulating the results to construct a receiver operating characteristic curve (ROC) for each of the shortlisted features, wherein an area under the curve (AUC) of the ROC is indicative of utility of the feature to distinguish between the training biological samples belonging to the first class and the second class.

4. The method (200) as claimed in claim 1, wherein calculating the model evaluation score (*MES*) comprises:

transforming the values of the set of features as follows:

$$F'_j = 0 \dots\dots\dots\dots\dots \text{ if } F_j < \widetilde{Q1}_J$$

$$F'_j = 1 \dots\dots\dots\dots\dots \text{ if } F_j > \widetilde{Q3}_J$$

$$F'_j = 0.5 \dots\dots\dots\dots\dots \text{ if } \widetilde{Q1}_J = \widetilde{Q3}_J$$

$$F'_j = \frac{F_j - \widetilde{Q1}_J}{\widetilde{Q3}_J - \widetilde{Q1}_J} \dots\dots\dots\dots\dots \text{ if } \widetilde{Q1}_J < F_j < \widetilde{Q3}_J;$$

collating the features out of the set of features as a set of numerator features ($F_{numerator}$) if $\widetilde{Q2_{BJ}} > \widetilde{Q2_{AJ}}$, else, collating the features out of the set of features as a set of denominator features ($F_{denominator}$);
constituting a ratio function for each of the candidate model as:

$$CM_K = \frac{\sum F_{numerator}}{\sum F_{denominator}}$$ ... when $F_{numerator} > 0$ and $F_{denominator} > 0$ or,

$$CM_K = \frac{\sum F_{numerator} + 1}{\sum F_{denominator} + 1}$$ ... when either $F_{numerator}$ or $F_{denominator} = 0$

wherein, $\sum F_{numerator}$ represents the sum of values of all numerator features for a particular training biological sample, and,
wherein, $\sum F_{denominator}$ represents the sum of values of all denominator features for a particular training biological sample;

generating a candidate model score ($CMS_K$) for each of the training biological samples in the internal train set;
removing the top 10 percentile and bottom 10 percentile scores as outliers from the set of scores $CMS_K$, and identifying maximum and minimum scores from the set $CMS_K$ as $CMS_{K_{max}}$ and $CMS_{K_{min}}$ respectively;

reclassifying the training biological samples in the internal train set by considering each score in the set $CMS_K$ as threshold, such that the training biological sample is classified into the second class B if $CMS_K$ is more than or equal to the threshold, or the training biological sample is classified into the first class if $CMS_K$ is less than the threshold;

calculating Matthew's correlation coefficients ($MCC$) for each of the thresholds based on a comparison of the reclassified training biological sample and the original classes of the training biological samples, to evaluate how well each of the thresholds are able to distinguish between the training biological samples associated to the first class and the second class;

identifying the threshold as a first threshold ($T_{max}$) which provides maximum absolute $MCC$ value;

discarding the candidate models for further evaluation if the maximum absolute $MCC$ value is less than 0.4;

considering the ($|MCC_{max}|$) value as the 'train-MCC' value ($MCC_{train}$) for the model $CM_K$ and the model and the first threshold ($T_{max}$) is used to classify the training biological samples in the internal-test set;

comparing the classification results on the training biological samples from the internal test set against the original classes of the training biological samples with pre-assigned labels, and the $MCC$ for the model $CM_K$ and the threshold $T_{max}$ threshold on the internal train set is calculated ($MCC_{test}$); and

calculating a model evaluation score *(MES)* for candidate model $CM_K$ as: $MES = |(MCC_{train} + MCC_{test})| - |(MCC_{train} - MC_{test})|$.

5. The method (200) as claimed in claim 1, further comprising evaluating collective classification efficiencies of the ensemble of forward models (ENS-$MD_{fwd}$) and the ensemble of reverse models (ENS-$MD_{rev}$), using an ensemble model scoring method, wherein a model scores ($MS$) corresponding to each of the ensemble is transformed into a scaled model scores ($SMS$) having values between -1 and +1, wherein,

$$SMS = (MS - T_{max})/( CMS_{K_{max}} - T_{max}), \ldots\ldots \text{ when } MS >= T_{max},$$

and

$$SMS = (MS - T_{max})/( T_{max} - CMS_{K_{min}} ), \ldots\ldots \text{ when } MS < T_{max},$$

wherein, $T_{max}$, $CMS_{K_{max}}$, and $CMS_{K_{min}}$ values corresponding to the respective model.

6. The method (200) of claim 5, further comprising calculating an average of all $SMS$ ($SMS_{avg}$) obtained using all models in the ensemble, wherein

$SMS_{avg} = SMS_{avg} * (+1)$ while using the ensemble of forward models (ENS-$MD_{fwd}$),

If $SMS_{avg} >= 0$, training biological sample is classified as the second class; and
If $SMS_{avg} < 0$, training biological sample is classified as the first class; and

$SMS_{avg} = SMS_{avg} * (-1)$ while using the ensemble of reverse model (ENS-$MD_{rev}$),

If $SMS_{avg} > 0$, training biological sample is classified as the second class; and
If $SMS_{avg} <= 0$, training biological sample is classified as the first class.

7. The method (200) as claimed in claim 6, further comprising selecting a final ensemble model ($FM_{ens}$) using the calculated $SMS_{avg}$, wherein the classification model is one of: the final single model ($FM_{single}$) or an ensemble of more than one classification models ($FM_{ens}$).

8. The method (200) as claimed in claim 1, wherein the first predefined criteria is if a feature ($F_j$) is observed to have significantly ($p<0.1$) different median values in the first class compared to the second class in >70% of predefined number of subsets, and if $\widetilde{Q2_{A_J}} >= Q2_{min}$ or $\widetilde{Q2_{B_J}} >= Q2_{min}$, $F_j$ is added to a set of shortlisted features ($SF$).

9. The method (200) as claimed in claim 1, wherein the relative abundance of each of the microbial taxonomic groups constituting the frugal set of markers, that are common to each of the minimum number of the multiplexed qPCR runs, is determined based on a normalizing factor associated with each multiplexed qPCR run and the relative abundance of associated microbial taxonomic group in the corresponding multiplexed qPCR run.

10. The method (200) as claimed in claim 1, wherein determining the relative abundance of each of the microbial taxonomic groups constituting the frugal set of markers in the test biological sample, comprises multiplying a ratio of inferred DNA concentrations of each marker of the frugal set of markers and an inferred DNA concentration of an anchor marker with the median abundance of the anchor marker across the plurality of training biological samples, wherein the anchor marker is selected from the frugal set of markers having a lowest variance in the associated relative abundance.

11. The method (200) as claimed in claim 1, wherein each train biological sample and the test biological sample are selected from a group comprising of a vaginal swab sample, a cervical mucus sample, a cervical swab sample, a vaginal swab including swab sample of a vaginal fornix, a urine sample, an amniotic fluid sample, a blood sample, a serum sample, a plasma sample, a placental swab, an umbilical swab, a stool sample, a skin swab, an oral swab, a saliva sample, a periodontal swab, a throat swab, a nasal swab, a vesicle fluid sample, a nasopharyngeal swab, a nares swab, a conjunctival swab, a genital swab, a rectum swab, a tracheal aspirate, and a bronchial swab.

12. The method (200) as claimed in claim 1, wherein one or more frugal markers from amongst the frugal set of markers that have a relatively higher median abundance or frequency of occurrence as compared to the median abundances or the frequency of occurrence of each frugal marker in the remaining frugal set of markers, across the plurality of training biological samples are common to the multiplex qPCR runs.

**Patentansprüche**

1. Verfahren (200) zum Identifizieren und Verwenden eines frugalen Satzes von Markern zur Klassifizierung einer biologischen Testprobe, wobei das Verfahren umfasst:

Sammeln einer Mehrzahl von biologischen Zugproben, umfassend eine erste Teilmenge von Proben, die einer ersten Klasse entsprechen, und eine zweite Teilmenge von Proben, die einer zweiten Klasse (202) entsprechen, wobei die erste Klasse zu Krankheits- oder Anomaliefällen gehört und die zweite Klasse zu gesunden Kontrollen gehört oder umgekehrt;
Extrahieren einer mikrobiellen Desoxyribonukleinsäure (DNA) aus jeder der Mehrzahl von biologischen Zugproben (204);
Sequenzieren der mikrobiellen DNA, die jeder der Mehrzahl von biologischen Zugproben zugeordnet ist, unter Verwendung eines Sequenzsierers, um DNA-Sequenzdaten zu erhalten, die jeder der Mehrzahl von biologischen Zugproben (206) entsprechen;
Analysieren, über einen oder mehrere Hardwareprozessoren, der DNA-Sequenzdaten, die jeder der Mehrzahl von biologischen Zugproben entsprechen, um eine Mehrzahl von mikrobiellen Häufigkeitsprofilen aus der Mehrzahl von biologischen Zugproben zu erzeugen, wobei jedes der Mehrzahl von mikrobiellen Häufigkeitsprofilen jeder der Mehrzahl von biologischen Zugproben entspricht und Häufigkeitswerte umfasst, die einer Mehrzahl von individuellen mikrobiellen taxonomischen Gruppen zugeordnet sind, die jeder der Mehrzahl von biologischen Zugproben (208) entsprechen;
Aufbauen, über den einen oder die mehreren Hardwareprozessoren, eines Ensemble-Klassifizierungsmodells durch Trainieren eines Maschinenlernmodells (ML-Modells) unter Verwendung der Mehrzahl von mikrobiellen Häufigkeitsprofilen, die der Mehrzahl von biologischen Zugproben zugeordnet sind, wobei das Ensemble-Klassifizierungsmodell einen Satz von nicht mehr als einer vordefinierten Anzahl von mikrobiellen taxonomischen Gruppen als den frugalen Satz von Markern umfasst, wobei die vordefinierte Anzahl von mikrobiellen taxonomischen Gruppen eine Teilmenge der Mehrzahl von individuellen mikrobiellen taxonomischen Gruppen ist, wobei die vordefinierte Anzahl von mikrobiellen taxonomischen Gruppen die Teilmenge von Markern ist, die 15 nicht überschreitet, und wobei das Ensemble-Klassifizierungsmodell ein oder mehrere Klassifizierungsteilmodelle umfasst und wobei das Ensemble-Klassifizierungsmodell konfiguriert ist, um die biologische Testprobe in eine der ersten Klasse und der zweiten Klasse (210) zu klassifizieren, und wobei das Ensemble-Klassifizierungsmodell aufgebaut wird durch:

(i) Zuweisen eines ersten Klassen-Tags oder eines zweiten Klassen-Tags zu jeder der Mehrzahl von biologischen Trainingsproben (302);
(ii) Erzeugen von Trainingsdaten, die eine Mehrzahl von mikrobiellen Häufigkeitsprofilen aus der Mehrzahl von biologischen Trainingsproben umfassen, wobei jedes mikrobielle Häufigkeitsprofile jeder der Mehrzahl von biologischen Trainingsproben entspricht und ein oder mehrere Merkmale und jeweilige Häufigkeitswerte umfasst, und wobei jedes Merkmal in dem zugeordneten mikrobiellen Häufigkeitsprofil einer von einer Mehrzahl von mikrobiellen taxonomischen Gruppen entspricht, die in der zugeordneten biologischen Trainingsprobe (304) vorhanden sind;

(iii) Aufteilen der Trainingsdaten in einen internen Trainingssatz und einen internen Testsatz, basierend auf einem vordefinierten ersten Parameter (306);

(iv) zufälliges Auswählen einer vordefinierten Anzahl von Teilmengen aus dem internen Trainingssatz basierend auf einem vordefinierten zweiten Parameter, wobei jede Teilmenge eine zufällig ausgewählte Mehrzahl von mikrobiellen Häufigkeitsprofilen umfasst, die den biologischen Trainingsproben in der zufällig ausgewählten Teilmenge entsprechen, und wobei jede Teilmenge einen verhältnismäßigen Teil von biologischen Trainingsproben umfasst, die zu der ersten Klasse gehören, und die verbleibenden biologischen Trainingsproben, die zu der zweiten Klasse gehören (308);

(v) Notieren, für jede ausgewählte Teilmenge, einer Verteilung der Häufigkeitswerte jedes der Merkmale über die Mehrzahl von biologischen Trainingsproben in der ausgewählten Teilmenge und der Verteilung der Häufigkeitswerte jedes der Merkmale über die biologischen Trainingsproben, die zu der ersten Klasse gehören, in der ausgewählten Teilmenge und die biologischen Trainingsproben, die zu der zweiten Klasse gehören, in der ausgewählten Teilmenge (310);

(vi) Berechnen, aus den notierten Verteilungen jeder ausgewählten Teilmenge, eines ersten Quartilwerts $Q1$ und eines dritten Quartilwerts $Q3$ der Verteilung jedes der Merkmale über jede der biologischen Trainingsproben in der ausgewählten Teilmenge (312);

(vii) Berechnen, für jede ausgewählte Teilmenge, eines zweiten Quartilwerts der Verteilung jedes der Merkmale über die biologischen Trainingsproben, die zu der ersten Klasse $Q2_A$ gehören, in der ausgewählten Teilmenge und die biologischen Trainingsproben, die zu der zweiten Klasse $Q2_B$ gehören, in der ausgewählten Teilmenge (314);

(viii) Berechnen von $Q1$, $Q3$, $Q2_A$ und $Q2_B$ für jede einer vordefinierten Anzahl von Teilmengen M (316);

(ix) Berechnen eines Medianwerts für jede der $Q1$, $Q3$, $Q2_A$ und $Q2_B$ (318);

(x) Durchführen eines Mann-Whitney-Tests, um zu prüfen, ob der Medianwert ($Q2_A$) des Merkmals in den biologischen Trainingsproben, die zu der ersten Klasse gehören, im Vergleich zu dem Medianwert ($Q2_B$) des zugehörigen Merkmals in den biologischen Trainingsproben, die zu der zweiten Klasse gehören, signifikant verschieden ist (320);

(xi) Kurzlisten der Merkmale basierend auf einem ersten vordefinierten Kriterium unter Verwendung berechneter Medianwerte und des Mann-Whitney-Tests (322);

(xii) Erzeugen eines Satzes von Merkmalen unter Verwendung der kurzgelisteten Merkmale unter Verwendung eines zweiten vordefinierten Kriteriums, wobei der Satz von Merkmalen kleiner oder gleich einem vordefinierten zweiten Kriteriumswert ist (324);

(xiii) Erzeugen einer Mehrzahl von Kombinationen der Merkmale, die in dem Satz von Merkmalen vorhanden sind, um eine Mehrzahl von Kandidatenmerkmalssätzen zu erzeugen, wobei eine Anzahl der Mehrzahl von Kombinationen der Merkmale gleich einem Minimum von zwei und einem Maximum des vordefinierten zweiten Kriteriumswerts ist (326);

(xiv) Aufbauen einer Mehrzahl von Kandidatenmodellen ($CM_K$), die jedem der Mehrzahl von Kandidatenmerkmalssätzen entsprechen (328);

(xv) Berechnen einer Modellevaluierungsbewertung ($MES$), die jedem der Mehrzahl von Kandidatenmodellen entspricht (330);

(xvi) Auswählen eines Modells mit einem höchsten $MES$ aus der Mehrzahl von Kandidatenmodellen als ein bestes Modell basierend auf einem ersten Schwellenwert ($T_{max}$), wobei das ausgewählte Modell als ein Vorwärtsmodell gekennzeichnet ist (332);

(xvii) Austauschen des ersten Klassen-Tags und des zweiten Klassen-Tags, die der ersten Klasse und der zweiten Klasse der Mehrzahl von biologischen Trainingsproben zugewiesen sind, die in den Trainingsdaten vorhanden sind (334);

(xviii) Identifizieren und anschließendes Kennzeichnen des Modells als ein umgekehrtes Modell durch Wiederholen der Schritte (ii) bis (xvi) für die Trainingsdaten, die nach dem Austauschen des ersten Klassen-Tags und des zweiten Klassen-Tags erhalten werden (336);

(xix) Erzeugen einer Mehrzahl von Vorwärtsmodellen und einer Mehrzahl von umgekehrten Modellen durch Wiederholen von Schritt (ii) bis (xviii) für eine vordefinierte Anzahl von Malen unter Verwendung von zufällig erzeugten Aufteilungen von internen Trainingssätzen und entsprechenden internen Testsätzen aus den Trainingsdaten (338);

(xx) Erzeugen eines Ensembles von Vorwärtsmodellen (ENS-$MD_{fwd}$) unter Verwendung der Mehrzahl von Vorwärtsmodellen und eines Ensembles von umgekehrten Modellen (ENS-$MD_{rev}$) unter Verwendung der Mehrzahl von umgekehrten Modellen (340);

(xxi) Identifizieren eines besten Vorwärtsmodells und eines besten umgekehrten Modells unter Verwendung der Modellevaluierungsbewertung (342); und

(xxii) Auswählen eines endgültigen Einzelmodells ($FM_{single}$) aus den besten Vorwärtsmodellen und dem besten umgekehrten Modell und eines endgültigen Ensemble-Klassifizierungsmodells ($FM_{ens}$) aus dem Ensemble von Vorwärtsmodellen und dem Ensemble von umgekehrten Modellen, basierend auf der Klassifikation der individuellen biologischen Trainingsproben aus den Trainingsdaten (344);

Entwerfen, über den einen oder die mehreren Hardwareprozessoren, eines Satzes von quantitativen Polymerasekettenreaktions(qPCR)-Sonden, die jeder mikrobiellen taxonomischen Gruppe entsprechen, die den frugalen Satz von Markern (212) bildet;

Bestimmen, über den einen oder die mehreren Hardwareprozessoren, einer Mindestanzahl von gemultiplexten qPCR-Läufen, die zum Quantifizieren einer relativen Häufigkeit jeder mikrobiellen taxonomischen Gruppe, die zu dem frugalen Satz von Markern gehört, erforderlich sind, basierend auf einer eindeutigen Anzahl von mikrobiellen taxonomischen Gruppen, die den frugalen Satz von Markern bilden, wobei jeder gemultiplexte qPCR-Lauf konfiguriert ist, um die relative Häufigkeit der vorbestimmten Teilmenge der mikrobiellen taxonomischen Gruppen, die den frugalen Satz von Markern bilden, in der biologischen Testprobe (214) zu bestimmen;

Bestimmen, über den einen oder die mehreren Hardwareprozessoren, einer Rangordnung jeder der mikrobiellen taxonomischen Gruppen, die den frugalen Satz von Markern bilden, basierend auf einem oder mehreren von (i) einer mittleren Häufigkeit jedes des frugalen Satzes von Markern aus der Mehrzahl von biologischen Trainingsproben und (ii) einer Häufigkeit des Auftretens jedes des frugalen Satzes von Markern über das eine oder die mehreren Klassifizierungsteilmodelle, die das Ensemble-Klassifizierungsmodell bilden, wobei die Rangordnung zum Bestimmen der Teilmenge von mikrobiellen taxonomischen Gruppen aus den taxonomischen Gruppen, die den frugalen Satz von Markern bilden, deren Häufigkeit zu sondieren ist, in der biologischen Testprobe unter Verwendung von mehr als einem gemultiplexten qPCR-Lauf (216) verwendet wird;

Bestimmen, über den einen oder die mehreren Hardwareprozessoren, der relativen Häufigkeit jeder der mikrobiellen taxonomischen Gruppen, die den frugalen Satz von Markern in der biologischen Testprobe bilden, durch Durchführen der bestimmten Mindestanzahl von gemultiplexten qPCR-Läufen unter Verwendung eines entworfenen Satzes von qPCR-Sonden und der Rangordnung jeder der mikrobiellen taxonomischen Gruppen, die den frugalen Satz von Markern (218) bilden; und

Klassifizieren, über den einen oder die mehreren Hardwareprozessoren, der biologischen Testprobe in eine der ersten Klasse und der zweiten Klasse unter Verwendung des Ensemble-Klassifizierungsmodells, basierend auf der relativen Häufigkeit jeder der mikrobiellen taxonomischen Gruppen, die den frugalen Satz von Markern in der biologischen Testprobe (220) bilden.

2. Verfahren (200) nach Anspruch 1, wobei die Mindestanzahl von gemultiplexten qPCR-Läufen unter Verwendung einer Formel bestimmt wird: $1 + \lceil (n-4)/4 \rceil$, wobei $n$ die eindeutige Anzahl von mikrobiellen taxonomischen Gruppen ist, die den frugalen Satz von Markern bilden.

3. Verfahren (200) nach Anspruch 1, ferner umfassend:

Klassifizieren jedes des Satzes von kurzgelisteten Merkmalen unter Verwendung eines zweiten Schwellenwertes, der sich von dem ersten Schwellenwert unterscheidet ($T_{max}$); und

Kumulieren der Ergebnisse, um eine Empfängerbetriebscharakteristikkurve (Receiver Operating Characteristic, ROC) für jedes der kurzgelisteten Merkmale zu konstruieren, wobei ein Bereich unter der Kurve (Area Under the Curve, AUC) der ROC die Nützlichkeit des Merkmals angibt, um zwischen den biologischen Trainingsproben zu unterscheiden, die zu der ersten Klasse und der zweiten Klasse gehören.

4. Verfahren (200) nach Anspruch 1, wobei das Berechnen der Modellevaluierungsbewertung *(MES)* umfasst:

Transformieren der Werte des Satzes von Merkmalen wie folgt:

$$F_j' = 0 \ldots\ldots\ldots\ldots\ldots \text{ wenn } F_j < \widetilde{Q1}_j$$

$$F_j' = 1 \quad \dots\dots\dots\dots \quad \text{wenn } F_j > \widetilde{Q3}_J$$

$$F_j' = 0.5 \quad \dots\dots\dots\dots \quad \text{wenn } \widetilde{Q1}_J = \widetilde{Q3}_J$$

$$F_j' = \frac{F_j - \widetilde{Q1}_J}{\widetilde{Q3}_J - \widetilde{Q1}_J} \quad \dots\dots\dots\dots \quad \text{wenn } \widetilde{Q1}_J < F_j < \widetilde{Q3}_J;$$

Zusammenstellen der Merkmale aus dem Satz von Merkmalen als einen Satz von Zählermerkmalen ($F_{numerator}$)

wenn $\widetilde{Q2_{B_J}} > \widetilde{Q2_{A_J}}$, andernfalls Zusammenstellen der Merkmale aus dem Satz von Merkmalen als einen Satz von Nennermerkmalen ($F_{denominator}$);

Bilden einer Verhältnisfunktion für jedes der Kandidatenmodelle als:

$$CM_K = \frac{\sum F_{Zähler}}{\sum F_{Nenner}} \quad \dots \text{ wenn } F_{Zähler} > 0 \text{ und } F_{Nenner} > 0 \text{ oder}$$

$$CM_K = \frac{\sum F_{Zähler} + 1}{\sum F_{Nenner} + 1} \quad \dots \text{ wenn entweder } F_{Zähler} \text{ oder } F_{Nenner} = 0$$

wobei $\sum F_{Zähler}$ die Summe von Werten aller Zählermerkmale für eine bestimmte biologische Trainingsprobe darstellt, und

wobei $\sum F_{Nenner}$ die Summe von Werten aller Denominatormerkmale für eine bestimmte biologische Trainingsprobe darstellt;

Erzeugen einer Kandidatenmodellbewertung ($CMS_K$) für jede der biologischen Trainingsproben in dem internen Trainingssatz;

Entfernen der oberen 10-Perzentil- und unteren 10-Perzentil-Bewertungen als Ausreißer aus dem Satz von Bewertungen $CMS_K$ und Identifizieren von maximalen und minimalen Bewertungen aus dem Satz $CMS_K$ als $CMS_{K_{max}}$ bzw. $CMS_{K_{min}}$;

Neuklassifizieren der biologischen Trainingsproben in dem internen Trainingssatz durch Berücksichtigen jeder Bewertung in dem Satz $CMS_K$ als Schwellenwert, so dass die biologische Trainingsprobe in die zweite Klasse B klassifiziert wird, wenn $CMS_K$ größer oder gleich dem Schwellenwert ist, oder die biologische Trainingsprobe in die erste Klasse klassifiziert wird, wenn $CMS_K$ kleiner als der Schwellenwert ist;

Berechnen von Matthew-Korrelationskoeffizienten ($MCC$) für jeden der Schwellenwerte basierend auf einem Vergleich der neuklassifizierten biologischen Trainingsprobe und der ursprünglichen Klassen der biologischen Trainingsproben, um zu bewerten, wie gut jeder der Schwellenwerte in der Lage ist, zwischen den biologischen Trainingsproben zu unterscheiden, die der ersten Klasse und der zweiten Klasse zugeordnet sind;

Identifizieren des Schwellenwerts als einen ersten Schwellenwert ($T_{max}$), der einen maximalen absoluten $MCC$ Wert bereitstellt;

Verwerfen der Kandidatenmodelle zur weiteren Bewertung, wenn der maximale absolute $MCC$ Wert kleiner als 0,4 ist;

Berücksichtigen des ($|MCC_{max}|$) Wertes als den 'Train-MCC'-Wert ($MCC_{train}$) für das Modell $CM_K$ und das Modell, und der erste Schwellenwert ($T_{max}$) wird verwendet, um die biologischen Trainingsproben in dem internen Testsatz zu klassifizieren;

Vergleichen der Klassifizierungsergebnisse an den biologischen Trainingsproben aus dem internen Testsatz mit den ursprünglichen Klassen der biologischen Trainingsproben mit vorab zugewiesenen Labels, und der $MCC$ für das Modell $CM_K$ und der Schwellenwert $T_{max}$ Schwellenwert an dem internen Trainingssatz wird berechnet ($MCC_{test}$); und

Berechnen einer Modellevaluierungsbewertung ($MES$) für das Kandidatenmodell $CM_K$ als: $MES = |(MCC_{train} + MCC_{test})| - |(MCC_{train} - MCC_{test})|$.

5. Verfahren (200) nach Anspruch 1, ferner umfassend das Bewerten kollektiver Klassifizierungseffizienzen des Ensembles von Vorwärtsmodellen (ENS-$MD_{fwd}$) und des Ensembles von umgekehrten Modellen (ENS-$MD_{rev}$) unter Verwendung eines Ensemble-Modellbewertungsverfahrens, wobei eine Modellbewertung (MS), die jedem der Ensembles entspricht, in eine skalierte Modellbewertung ($SMS$) mit Werten zwischen -1 und +1 transformiert wird, wobei

$$SMS = (MS - T_{max})/(CMS_{K_{max}} - T_{max}), .... \text{ wenn } MS >= T_{max},$$

und

$$SMS = (MS - T_{max})/(T_{max} - CMS_{K_{min}}), .... \text{ wenn } MS < T_{max},$$

wobei $T_{max}$, $CMS_{K_{max}}$ und $CMS_{K_{min}}$ Werte dem jeweiligen Modell entsprechen.

6. Verfahren (200) nach Anspruch 5, ferner umfassend das Berechnen eines Durchschnitts aller $SMS(SMS_{avg})$, die unter Verwendung aller Modelle in dem Ensemble erhalten werden, wobei
$SMS_{avg} = SMS_{avg} * (+1)$, während das Ensemble von Vorwärtsmodellen (ENS-$MD_{fwd}$) verwendet wird,

wenn $SMS_{avg} >= 0$, wird die biologische Trainingsprobe als die zweite Klasse klassifiziert; und
wenn $SMS_{avg} < 0$, wird die biologische Trainingsprobe als die erste Klasse klassifiziert; und

$SMS_{avg} = SMS_{avg} * (-1)$, während das Ensemble von umgekehrten Modellen (ENS-$MD_{rev}$) verwendet wird,

wenn $SMS_{avg} > 0$, wird die biologische Trainingsprobe als die zweite Klasse klassifiziert; und
wenn $SMS_{avg} <= 0$, wird die biologische Trainingsprobe als die erste Klasse klassifiziert.

7. Verfahren (200) nach Anspruch 6, ferner umfassend das Auswählen eines endgültigen Ensemble-Modells ($FM_{ens}$) unter Verwendung des berechneten $SMS_{avg}$, wobei das Klassifizierungsmodell eines ist von: dem endgültigen Einzelmodell ($FM_{single}$) oder einem Ensemble von mehr als einem Klassifizierungsmodell ($FM_{ens}$).

8. Verfahren (200) nach Anspruch 1, wobei das erste vordefinierte Kriterium ist, wenn festgestellt wird, dass ein Merkmal ($F_j$) signifikant (p < 0,1) verschiedene Medianwerte in der ersten Klasse im Vergleich zu der zweiten Klasse in >70 % der vordefinierten Anzahl von Teilmengen aufweist, und wenn $\widetilde{Q2_{A_J}} >= Q2_{min}$ oder $\widetilde{Q2_{B_J}} >= Q2_{min}$, $F_j$ zu einem Satz von kurzgelisteten Merkmalen hinzugefügt wird ($SF$).

9. Verfahren (200) nach Anspruch 1, wobei die relative Häufigkeit jeder der mikrobiellen taxonomischen Gruppen, die den frugalen Satz von Markern bilden, die jedem der Mindestanzahl der gemultiplexten qPCR-Läufe gemeinsam sind, basierend auf einem Normalisierungsfaktor, der jedem gemultiplexten qPCR-Lauf zugeordnet ist, und der relativen Häufigkeit der zugeordneten mikrobiellen taxonomischen Gruppe in dem entsprechenden gemultiplexten qPCR-Lauf bestimmt wird.

10. Verfahren (200) nach Anspruch 1, wobei das Bestimmen der relativen Häufigkeit jeder der mikrobiellen taxonomischen Gruppen, die den frugalen Satz von Markern in der biologischen Testprobe bilden, das Multiplizieren eines Verhältnisses von abgeleiteten DNA-Konzentrationen jedes Markers des frugalen Satzes von Markern und einer abgeleiteten DNA-Konzentration eines Ankermarkers mit der mittleren Häufigkeit des Ankermarkers über die Mehrzahl von biologischen Trainingsproben umfasst, wobei der Ankermarker aus dem frugalen Satz von Markern mit einer geringsten Varianz in der zugeordneten relativen Häufigkeit ausgewählt wird.

11. Verfahren (200) nach Anspruch 1, wobei jede biologische Trainingsprobe und die biologische Testprobe aus einer Gruppe ausgewählt werden, die eine vaginale Abstrichprobe, eine zervikale Schleimprobe, eine zervikale Abstrichprobe, einen vaginalen Abstrich, der eine Abstrichprobe eines vaginalen Fornix umfasst, eine Urinprobe, eine Fruchtwasserprobe, eine Blutprobe, eine Serumprobe, eine Plasmaprobe, einen Plazentaabstrich, einen Nabelschnurabstrich, eine Stuhlprobe, einen Hautabstrich, einen oralen Abstrich, eine Speichelprobe, einen Periodontalabstrich, einen Rachenabstrich, einen Nasenabstrich, eine Vesikelflüssigkeitsprobe, einen Nasopharynxabstrich, einen Nasenlochabstrich, einen Bindehautabstrich, einen Genitalabstrich, einen Rektumabstrich, ein Trachealaspirat und einen Bronchialabstrich umfasst.

12. Verfahren (200) nach Anspruch 1, wobei ein oder mehrere frugale Marker aus dem frugalen Satz von Markern, die eine relativ höhere mittlere Häufigkeit oder Häufigkeit des Auftretens im Vergleich zu den mittleren Häufigkeiten oder der Häufigkeit des Auftretens jedes frugalen Markers in dem verbleibenden frugalen Satz von Markern aufweisen, über die Mehrzahl von biologischen Trainingsproben den gemultiplexten qPCR-Läufen gemeinsam sind.

**Revendications**

1. Procédé (200) d'identification et d'utilisation d'un ensemble frugal de marqueurs pour la classification d'un échantillon biologique d'essai, le procédé comprenant :

la collecte d'une pluralité d'échantillons biologiques d'entraînement comprenant un premier sous-ensemble d'échantillons correspondant à une première classe et un second sous-ensemble d'échantillons correspondant à une seconde classe (202), dans lequel la première classe appartient à des cas malades ou d'anomalies et la seconde classe appartient à des témoins sains ou vice versa ;

l'extraction d'un acide désoxyribonucléique (ADN) microbien à partir de chacun de la pluralité d'échantillons biologiques d'entraînement (204) ;

le séquençage de l'ADN microbien associé à chacun de la pluralité d'échantillons biologiques d'entraînement, à l'aide d'un séquenceur, pour obtenir des données de séquence d'ADN correspondant à chacun de la pluralité d'échantillons biologiques d'entraînement (206) ;

l'analyse, via un ou plusieurs processeurs matériels, des données de séquence d'ADN correspondant à chacun de la pluralité d'échantillons biologiques d'entraînement, pour générer une pluralité de profils d'abondance microbienne à partir de la pluralité d'échantillons biologiques d'entraînement, dans lequel chacun de la pluralité de profils d'abondance microbienne correspond à chacun de la pluralité d'échantillons biologiques d'entraînement et comprend des valeurs d'abondance associées à une pluralité de groupes taxonomiques microbiens individuels correspondant à chacun de la pluralité d'échantillons biologiques d'entraînement (208) ;

la construction, via les un ou plusieurs processeurs matériels, d'un modèle de classification d'ensemble par entraînement d'un modèle d'apprentissage automatique (ML) à l'aide de la pluralité de profils d'abondance microbienne associés à la pluralité d'échantillons biologiques d'entraînement, dans lequel le modèle de classification d'ensemble comprend un ensemble de pas plus d'un nombre prédéfini de groupes taxonomiques microbiens en tant qu'ensemble frugal de marqueurs, dans lequel le nombre prédéfini de groupes taxonomiques microbiens est un sous-ensemble de la pluralité de groupes taxonomiques microbiens individuels, dans lequel le nombre prédéfini de groupes taxonomiques microbiens est le sous-ensemble de marqueurs ne dépassant pas 15, et dans lequel le modèle de classification d'ensemble comprend un ou plusieurs sous-modèles de classification et dans lequel le modèle de classification d'ensemble est configuré pour classifier l'échantillon biologique d'essai dans l'une de la première classe et de la seconde classe (210), et dans lequel le modèle de classification d'ensemble est construit par :

(i) l'attribution d'une étiquette de première classe ou d'une étiquette de seconde classe à chacun de la pluralité d'échantillons biologiques d'entraînement (302) ;

(ii) la génération de données d'entraînement comprenant une pluralité de profils d'abondance microbienne à partir de la pluralité d'échantillons biologiques d'entraînement, dans lequel chaque profil d'abondance microbienne correspond à chacun de la pluralité d'échantillons biologiques d'entraînement et comprend une ou plusieurs caractéristiques et des valeurs d'abondance respectives, et dans lequel chaque caractéristique dans le profil d'abondance microbienne associé correspond à l'un d'une pluralité de groupes taxonomiques microbiens présents dans l'échantillon biologique d'entraînement associé (304) ;

(iii) la division des données d'entraînement en un ensemble d'entraînement interne et un ensemble d'essai interne, sur la base d'un premier paramètre prédéfini (306) ;

(iv) la sélection aléatoire d'un nombre prédéfini de sous-ensembles parmi l'ensemble d'entraînement interne sur la base d'un second paramètre prédéfini, dans lequel chaque sous-ensemble comprend une pluralité sélectionnée aléatoirement de profils d'abondance microbienne correspondant aux échantillons biologiques d'entraînement dans le sous-ensemble sélectionné aléatoirement, et dans lequel chaque sous-ensemble comprend une partie proportionnelle d'échantillons biologiques d'entraînement appartenant à la première classe et les échantillons biologiques d'entraînement restants appartenant à la seconde classe (308) ;

(v) la note, pour chaque sous-ensemble sélectionné, d'une distribution des valeurs d'abondance de chacune des caractéristiques à travers la pluralité d'échantillons biologiques d'entraînement dans le sous-ensemble sélectionné, et de la distribution des valeurs d'abondance de chacune des caractéristiques à travers les échantillons biologiques d'entraînement appartenant à la première classe dans le sous-ensemble sélectionné et les échantillons biologiques d'entraînement appartenant à la seconde classe dans le sous-ensemble sélectionné (310) ;

(vi) le calcul, à partir des distributions notées de chaque sous-ensemble sélectionné, d'une valeur de premier quartile $Q1$ et d'une valeur de troisième quartile $Q3$ de la distribution de chacune des caractéristiques à travers chacun des échantillons biologiques d'entraînement dans le sous-ensemble sélectionné (312) ;

(vii) le calcul, pour chaque sous-ensemble sélectionné, d'une valeur de deuxième quartile de la distribution de chacune des caractéristiques à travers les échantillons biologiques d'entraînement appartenant à la première classe $Q2_A$ dans le sous-ensemble sélectionné et les échantillons biologiques d'entraînement appartenant à la seconde classe $Q2_B$ dans le sous-ensemble sélectionné (314) ;

(viii) le calcul $Q1$, $Q3$, $Q2_A$ et $Q2_B$ pour chacun d'un nombre prédéfini de sous-ensembles $M$ (316) ;

(ix) le calcul d'une valeur médiane pour chacun des $Q1$, $Q3$, $Q2_A$ et $Q2_B$ (318)

(x) la réalisation d'un test de Mann-Whitney pour vérifier si la valeur médiane ($Q2_A$) de la caractéristique dans les échantillons biologiques d'entraînement appartenant à la première classe est significativement différente par rapport à la valeur médiane ($Q2_B$) de la caractéristique associée dans les échantillons biologiques d'entraînement appartenant à la seconde classe (320) ;

(xi) la présélection des caractéristiques sur la base d'un premier critère prédéfini en utilisant des valeurs médianes calculées et le test de Mann-Whitney (322) ;

(xii) la génération d'un ensemble de caractéristiques en utilisant les caractéristiques présélectionnées en utilisant un deuxième critère prédéfini, dans lequel l'ensemble de caractéristiques est inférieur ou égal à une deuxième valeur de critère prédéfinie (324) ;

(xiii) la création d'une pluralité de combinaisons des caractéristiques présentes dans l'ensemble de caractéristiques pour générer une pluralité d'ensembles de caractéristiques candidats, dans lequel un nombre de la pluralité de combinaisons des caractéristiques est égal à un minimum de deux et un maximum de la deuxième valeur de critère prédéfinie (326) ;

(xiv) la construction d'une pluralité de modèles candidats ($CM_K$) correspondant à chacun de la pluralité d'ensembles de caractéristiques candidats (328) ;

(xv) le calcul d'un score d'évaluation de modèle (MES) correspondant à chacun de la pluralité de modèles candidats (330) ;

(xvi) la sélection d'un modèle ayant un MES le plus élevé, parmi la pluralité de modèles candidats en tant que meilleur modèle, sur la base d'un premier seuil ($T_{max}$), dans lequel le modèle sélectionné est étiqueté en tant que modèle avant (332) ;

(xvii) la permutation de l'étiquette de première classe et de l'étiquette de seconde classe attribuées à la première classe et à la seconde classe de la pluralité d'échantillons biologiques d'entraînement présents dans les données d'entraînement (334) ;

(xviii) l'identification et ensuite l'étiquetage du modèle en tant que modèle inverse en répétant les étapes (ii) à (xvi) pour les données d'entraînement obtenues après la permutation de l'étiquette de première classe et de l'étiquette de seconde classe (336) ;

(xix) la génération d'une pluralité de modèles avant et d'une pluralité de modèles inverses en répétant les étapes (ii) à (xviii) pendant un nombre prédéfini de fois en utilisant des divisions créées aléatoirement d'ensembles d'entraînement internes et d'ensembles d'essai internes correspondants à partir des données d'entraînement (338) ;

(xx) la génération d'un ensemble de modèles avant ($ENS\text{-}MD_{fwd}$) en utilisant la pluralité de modèles avant et d'un ensemble de modèles inverses (ENS-$MD_{rev}$) en utilisant la pluralité de modèles inverses (340) ;

(xxi) l'identification d'un meilleur modèle avant et d'un meilleur modèle inverse en utilisant le score d'évaluation de modèle (342) ; et

(xxii) le choix d'un modèle unique final ($FM_{unique}$) parmi les meilleurs modèles avant et le meilleur modèle inverse, et d'un modèle de classification d'ensemble final ($FM_{ens}$) parmi l'ensemble de modèles avant et l'ensemble de modèles inverses, sur la base de la classification des échantillons biologiques d'entraînement individuels à partir des données d'entraînement (344) ;

la conception, via les un ou plusieurs processeurs matériels, d'un ensemble de sondes de réaction en chaîne par polymérase quantitative (qPCR) correspondant à chaque groupe taxonomique microbien constituant l'ensemble frugal de marqueurs (212) ;

la détermination, via les un ou plusieurs processeurs matériels, d'un nombre minimum de cycles qPCR multiplexés requis pour quantifier une abondance relative de chaque groupe taxonomique microbien appartenant à l'ensemble frugal de marqueurs, sur la base d'un nombre unique de groupes taxonomiques microbiens constituant l'ensemble frugal de marqueurs, dans lequel chaque cycle qPCR multiplexé est configuré pour déterminer l'abondance relative du sous-ensemble prédéterminé des groupes taxonomiques microbiens constituant l'ensemble frugal de marqueurs, dans l'échantillon biologique d'essai (214) ;

la détermination, via les un ou plusieurs processeurs matériels, d'un classement de chacun des groupes taxonomiques microbiens constituant l'ensemble frugal de marqueurs, sur la base d'un ou plusieurs parmi (i) une abondance médiane de chacun de l'ensemble frugal de marqueurs à partir de la pluralité d'échantillons biologiques d'entraînement, et (ii) une fréquence d'occurrence de chacun de l'ensemble frugal de marqueurs à travers les un ou plusieurs sous-modèles de classification constituant le modèle de classification d'ensemble, dans lequel le classement est utilisé pour déterminer le sous-ensemble de groupes taxonomiques microbiens parmi les groupes taxonomiques constituant l'ensemble frugal de marqueurs dont l'abondance doit être sondée, dans l'échantillon biologique d'essai, en utilisant plus d'un cycle qPCR multiplexé (216) ;

la détermination, via les un ou plusieurs processeurs matériels, de l'abondance relative de chacun des groupes taxonomiques microbiens constituant l'ensemble frugal de marqueurs dans l'échantillon biologique d'essai, en effectuant le nombre minimum déterminé de cycles qPCR multiplexés en utilisant un ensemble conçu de sondes qPCR, et le classement de chacun des groupes taxonomiques microbiens constituant l'ensemble frugal de marqueurs (218) ; et

la classification, via les un ou plusieurs processeurs matériels, de l'échantillon biologique d'essai dans l'une de la première classe et de la seconde classe, en utilisant le modèle de classification d'ensemble, sur la base de l'abondance relative de chacun des groupes taxonomiques microbiens constituant l'ensemble frugal de marqueurs dans l'échantillon biologique d'essai (220).

2. Procédé (200) selon la revendication 1, dans lequel le nombre minimum de cycles qPCR multiplexés est déterminé en utilisant une formule : $1 + \lceil (n - 4)/4 \rceil$, où n est le nombre unique de groupes taxonomiques microbiens constituant l'ensemble frugal de marqueurs.

3. Procédé (200) selon la revendication 1, comprenant en outre :

la classification de chacune de l'ensemble de caractéristiques présélectionnées en utilisant une deuxième valeur de seuil différente du premier seuil ($T_{max}$) ; et

la cumulation des résultats pour construire une courbe de caractéristique de fonctionnement de récepteur (ROC) pour chacune des caractéristiques présélectionnées, dans lequel une zone sous la courbe (AUC) de la ROC est indicative de l'utilité de la caractéristique pour faire la distinction entre les échantillons biologiques d'entraînement appartenant à la première classe et la seconde classe.

4. Procédé (200) selon la revendication 1, dans lequel le calcul du score d'évaluation de modèle *(MES)* comprend :

la transformation des valeurs de l'ensemble de caractéristiques comme suit :

$$F_j' = 0 \dots\dots\dots\dots\dots \text{ si } F_j < \widetilde{Q1}_J$$

$$F_j' = 1 \dots\dots\dots\dots\dots \text{ si } F_j > \widetilde{Q3}_J$$

$$F_j' = 0.5 \dots\dots\dots\dots\dots \text{ si } \widetilde{Q1}_J = \widetilde{Q3}_J$$

$$F_j' = \frac{F_j - \widetilde{Q1}_J}{\widetilde{Q3}_J - \widetilde{Q1}_J} \dots\dots\dots\dots\dots \text{ si } \widetilde{Q1}_J < F_j < \widetilde{Q3}_J \text{ ;}$$

la compilation des caractéristiques de l'ensemble de caractéristiques comme un ensemble de caractéristiques numératrices ($F_{numératrice}$) si $\widetilde{Q2_{B_J}} > \widetilde{Q2_{A_J}}$, sinon, la compilation des caractéristiques de l'ensemble de caractéristiques comme un ensemble de caractéristiques dénominatrices ($F_{dénominatrice}$) ;

la constitution d'une fonction de rapport pour chacun des modèles candidats comme

$$CM_K = \frac{\sum F_{numératrice}}{\sum F_{dénominatrice}} \text{ ... lorsque } F_{numératrice} > 0 \text{ et } F_{dénominatrice} > 0 \text{ ou,}$$

$$CM_K = \frac{\sum F_{numératrice} + 1}{\sum F_{dénominatrice} + 1} \text{ ... lorsque soit } F_{numératrice} \text{ soit } F_{dénominatrice} = 0$$

dans lequel, $\sum F_{numératrice}$ représente la somme de valeurs de toutes les caractéristiques numératrices pour un échantillon biologique d'entraînement particulier, et,

dans lequel, $\sum F_{dénominatrice}$ représente la somme de valeurs de toutes les caractéristiques dénominatrices pour un échantillon biologique d'entraînement particulier ;

la génération d'un score de modèle candidat ($CMS_K$) pour chacun des échantillons biologiques d'entraînement dans l'ensemble d'entraînement interne ;

la suppression des scores du 10e centile supérieur et du 10e centile inférieur comme valeurs aberrantes de l'ensemble de scores $CMS_K$, et l'identification de scores maximum et minimum de l'ensemble $CMS_K$ comme $CMS_{Kmax}$ et $CMS_{Kmin}$ respectivement ;

la reclassification des échantillons biologiques d'entraînement dans l'ensemble d'entraînement interne en considérant chaque score dans l'ensemble $CMS_K$ comme seuil, de sorte que l'échantillon biologique d'entraînement est classé dans la seconde classe B si $CMS_K$ est supérieur ou égal au seuil, ou l'échantillon biologique d'entraînement est classé dans la première classe si $CMS_K$ est inférieur au seuil ;

le calcul de coefficients de corrélation de Matthew ($MCC$) pour chacun des seuils sur la base d'une comparaison de l'échantillon biologique d'entraînement reclassé et des classes d'origine des échantillons biologiques d'entraînement, pour évaluer dans quelle mesure chacun des seuils est capable de faire la distinction entre les échantillons biologiques d'entraînement associés à la première classe et à la seconde classe ;

l'identification du seuil comme un premier seuil ($T_{max}$) qui fournit une valeur absolue maximale $MCC$;

le rejet des modèles candidats pour une évaluation supplémentaire si la valeur absolue maximale $MCC$ est inférieure à 0,4 ;

la considération de la valeur ($|MCC_{max}|$) comme la valeur « train-MCC » ($MCC_{train}$) pour le modèle $CM_K$ et le modèle et le premier seuil ($T_{max}$) est utilisé pour classer les échantillons biologiques d'entraînement dans l'ensemble d'essai interne ;

la comparaison des résultats de classification sur les échantillons biologiques d'entraînement provenant de l'ensemble d'essai interne par rapport aux classes d'origine des échantillons biologiques d'entraînement avec des étiquettes pré-attribuées, et la valeur $MCC$ pour le modèle $CM_K$ et le seuil $T_{max}$ sur l'ensemble d'entraînement interne est calculé ($MCC_{test}$) ; et

le calcul d'un score d'évaluation de modèle *(MES)* pour le modèle candidat $CM_K$ comme : $MES = |(MCC_{train} + MCC_{test})| - |(MCC_{train} - MCC_{test})|$.

5. Procédé (200) selon la revendication 1, comprenant en outre l'évaluation d'efficacités de classification collective de l'ensemble de modèles avant (ENS-$MD_{fwd}$) et de l'ensemble de modèles inverses (ENS-$MD_{rev}$), en utilisant un procédé de notation de modèle d'ensemble, dans lequel un score de modèle (MS) correspondant à chacun de l'ensemble est transformé en un score de modèle mis à l'échelle (*SMS*) ayant des valeurs entre -1 et +1, dans lequel,

$$SMS = (MS - T_{max})/(CMS_{K_{max}} - T_{max}), \dots \text{ lorsque } MS >= T_{max},$$

et

$$SMS = (MS - T_{max})/(T_{max} - CMS_{K_{min}}), \dots \text{ lorsque } MS < T_{max},$$

dans lequel les valeurs $T_{max}$, $CMS_{K_{max}}$, et $CMS_{K_{min}}$ correspondent au modèle respectif.

6. Procédé (200) selon la revendication 5, comprenant en outre le calcul d'une moyenne de tous les $SMS$($SMS_{avg}$) obtenus en utilisant tous les modèles dans l'ensemble, dans lequel $SMS_{avg} = SMS_{avg} * (+1)$ tout en utilisant l'ensemble de modèles avant (ENS-$MD_{fwd}$),

Si $SMS_{avg} >= 0$, l'échantillon biologique d'entraînement est classé comme la seconde classe ; et
Si $SMS_{avg} < 0$, l'échantillon biologique d'entraînement est classé comme la première classe ; et

$SMS_{avg} = SMS_{avg} * (-1)$ tout en utilisant l'ensemble de modèles inverses (ENS-$MD_{rev}$),

Si $SMS_{avg} > 0$, l'échantillon biologique d'entraînement est classé comme la seconde classe ; et
Si $SMS_{avg} <= 0$, l'échantillon biologique d'entraînement est classé comme la première classe.

7. Procédé (200) selon la revendication 6, comprenant en outre la sélection d'un modèle d'ensemble final ($FM_{ens}$) en utilisant le $SMS_{avg}$ calculé, dans lequel le modèle de classification est l'un parmi : le modèle unique final ($FM_{unique}$) ou un ensemble de plus d'un modèle de classification ($FM_{ens}$).

8. Procédé (200) selon la revendication 1, dans lequel le premier critère prédéfini est si une caractéristique ($F_j$) est

**EP 4 451 285 B1**

observée comme ayant significativement (p < 0,1) des valeurs médianes différentes dans la première classe par rapport à la seconde classe dans > 70 % du nombre prédéfini de sous-ensembles, et si $\widetilde{Q2_{A_J}} >= Q2_{min}$ ou $\widetilde{Q2_{B_J}} >= Q2_{min}$, $F_j$ est ajouté à un ensemble de caractéristiques présélectionnées (SF).

9. Procédé (200) selon la revendication 1, dans lequel l'abondance relative de chacun des groupes taxonomiques microbiens constituant l'ensemble frugal de marqueurs, qui sont communs à chacun du nombre minimum de cycles qPCR multiplexés, est déterminée sur la base d'un facteur de normalisation associé à chaque cycle qPCR multiplexé et de l'abondance relative du groupe taxonomique microbien associé dans le cycle qPCR multiplexé correspondant.

10. Procédé (200) selon la revendication 1, dans lequel la détermination de l'abondance relative de chacun des groupes taxonomiques microbiens constituant l'ensemble frugal de marqueurs dans l'échantillon biologique d'essai, comprend la multiplication d'un rapport de concentrations d'ADN déduites de chaque marqueur de l'ensemble frugal de marqueurs et d'une concentration d'ADN déduite d'un marqueur d'ancrage par l'abondance médiane du marqueur d'ancrage à travers la pluralité d'échantillons biologiques d'entraînement, dans lequel le marqueur d'ancrage est sélectionné parmi l'ensemble frugal de marqueurs ayant une variance la plus faible dans l'abondance relative associée.

11. Procédé (200) selon la revendication 1, dans lequel chaque échantillon biologique d'entraînement et l'échantillon biologique d'essai sont sélectionnés dans un groupe comprenant un échantillon d'écouvillon vaginal, un échantillon de mucus cervical, un échantillon d'écouvillon cervical, un écouvillon vaginal comprenant un échantillon d'écouvillon d'un fornix vaginal, un échantillon d'urine, un échantillon de liquide amniotique, un échantillon de sang, un échantillon de sérum, un échantillon de plasma, un écouvillon placentaire, un écouvillon ombilical, un échantillon de selles, un écouvillon de peau, un écouvillon oral, un échantillon de salive, un écouvillon parodontal, un écouvillon de gorge, un écouvillon nasal, un échantillon de fluide vésiculaire, un écouvillon nasopharyngé, un écouvillon nasal, un écouvillon conjonctival, un écouvillon génital, un écouvillon rectum, un aspirat trachéal, et un écouvillon bronchique.

12. Procédé (200) selon la revendication 1, dans lequel un ou plusieurs marqueurs frugaux parmi l'ensemble frugal de marqueurs qui ont une abondance médiane relativement plus élevée ou une fréquence d'occurrence par rapport aux abondances médianes ou à la fréquence d'occurrence de chaque marqueur frugal dans l'ensemble frugal de marqueurs restant, à travers la pluralité d'échantillons biologiques d'entraînement, sont communs aux cycles qPCR multiplexés.

100

Recommendation
module **114**

Sample
collection
module **102**

DNA extractor
**104**

Sequencer
**106**

ML model
generation
module **112**

One or more
hardware
processors **110**

Memory
**108**

FIG. 1

200

Collecting a plurality of train biological samples comprising of a first subset of samples corresponding to a first class and a second subset of samples corresponding to a second class **202**

Extracting a microbial Deoxyribonucleic Acid (DNA) from each of the plurality of train biological samples **204**

Sequencing the microbial DNA associated with each of the plurality of train biological samples, using a sequencer, to obtain a DNA sequence data corresponding to each of the plurality of train biological samples **206**

Analyzing the DNA sequence data corresponding to each of the plurality of train biological samples, to generate a plurality of microbial abundance profiles from the plurality of train biological samples, wherein each microbial abundance profile corresponds to each of the plurality of train biological samples and comprises of abundance values associated with a plurality of individual microbial taxonomic groups corresponding to each of the plurality of train biological samples **208**

Building an ensemble classification model by training a machine learning (ML) model using the plurality of microbial abundance profiles associated with the plurality of train biological samples, wherein the ensemble classification model comprises of a set of no more than a predefined number of microbial taxonomic groups as the frugal set of markers, wherein the set of no more than the predefined number of microbial taxonomic groups is a subset of the plurality of individual microbial taxonomic groups, and wherein the ensemble classification model comprises of one or more classification sub-models and is configured to classify the test biological sample to one of the first class and the second class **210**

A

FIG. 2A

200

( A )

Designing a set of qPCR-probes corresponding to each microbial taxonomic group constituting to the frugal set of markers **212**

Determining a minimum number of multiplexed qPCR runs required for quantifying a relative abundance of each microbial taxonomic group belonging to the frugal set of markers, based on a unique number of microbial taxonomic groups constituting the frugal set of markers, wherein each multiplexed qPCR run is configured to determine the relative abundance of the predetermined subset of the microbial taxonomic groups constituting the frugal set of markers, in the test biological sample **214**

Determining a ranking of each of the microbial taxonomic groups constituting the frugal set of markers, based on one or more of (i) a median abundance of each of the frugal set of markers from the plurality of train biological samples, and (ii) a frequency of occurrence of each of the frugal set of markers across the one or more classification sub-models constituting the ensemble classification model, wherein the ranking is utilized for determining the subset of microbial taxonomic groups from amongst the taxonomic groups constituting the frugal set of markers whose abundance is to be probed, in the test biological sample, using more than one multiplexed qPCR runs **216**

Determining the relative abundance of each of the microbial taxonomic groups constituting the frugal set of markers in the test biological sample, by performing the determined minimum number of multiplexed qPCR runs utilizing a designed set of qPCR probes, and the ranking of each of the microbial taxonomic groups constituting the frugal set of markers **218**

Classifying the test biological sample to one of the first class and the second class, utilizing the ensemble classification model, based on the relative abundance of each of the microbial taxonomic groups constituting the frugal set of markers in the test biological sample **220**

FIG. 2B

/ 300

Assigning one of a first class tag or a second class tag to each of the plurality of training biological samples **302**

Generating a training data comprising a plurality of microbial abundance profiles from the plurality of training biological samples, wherein each microbial abundance profile corresponds to each of the plurality of training biological samples and comprises of one or more features and respective abundance values, and wherein each feature in the associated microbial abundance profile corresponds to one of a plurality of microbial taxonomic groups present in the associated training biological sample **304**

Partitioning the training data into an internal training set and an internal test set, based on a predefined first parameter **306**

Randomly selecting a predefined number of subsets out of the internal training set based on a predefined second parameter, wherein each subset comprises of a randomly selected plurality of microbial abundance profiles corresponding to the training biological samples in the randomly selected subset, and wherein each subset comprises a proportionate part of training biological samples belonging to the first class and the remaining training biological samples belonging to the second class **308**

Noting, for each selected subset, a distribution of the abundance values of each of the features across the plurality of training biological samples in the selected subset, and the distribution of the abundance values of each of the features across the training biological samples belonging to the first class in the selected subset and the training biological samples belonging to the second class in the selected subset **310**

Calculating, from the noted distributions of each selected subset, a first quartile value Q1 and a third quartile value Q3 of the distribution of each of the features across each of the plurality of training biological samples in the selected subset **312**

( B )

FIG. 3A

B

Calculating, for each selected subset, a second quartile value of the distribution of each of the features across the training biological samples belonging to the first class in the selected subset and the training biological samples belonging to the second class in the selected subset **314**

Calculating Q1, Q3, Q2A and Q2B for each of a predefined number of subsets M **316**

Calculating a median value for each of the Q1, Q3, Q2A and Q2B **318**

Performing a Mann-Whitney test to check whether the median value (Q2A) of the feature in the training biological samples belonging to the first class is significantly different as compared to the median value (Q2B) of the associated feature in the training biological samples belonging to the second class **320**

Shortlisting the features based on a first predefined criteria utilizing calculated median values and the Mann-Whitney test **322**

Generating a set of features using the shortlisted features using a second predefined criteria, wherein the set of features are less than or equal to a predefined second criteria value **324**

Creating a plurality of combinations of the features present in the set of features to generate a plurality of candidate feature sets, wherein a number of the plurality of combinations of the features is equal to a minimum of two and a maximum of the predefined second criteria value **326**

C

FIG. 3B

C

Building a plurality of candidate models corresponding to each of the plurality of candidate feature sets **328**

Calculating a model evaluation score (MES) corresponding to each of the plurality of candidate models **330**

Selecting a model having a highest MES, out of the plurality of candidate models as a best model, based on a first threshold, wherein the selected model is tagged as a forward model **332**

Swapping the first class tag and the second class tag assigned to the first class and the second class of the plurality of training biological samples present in the training data **334**

Identifying the model as a reverse model by repeating the steps (**304**) through (**332**) for the training data obtained after swapping the first class tag and the second class tag **336**

generating a plurality of forward models and a plurality of reverse models by repeating step (**304**) through (**336**) for a predefined number of times using randomly created partitions of internal training sets and corresponding internal test sets from the training data **338**

Generating an ensemble of forward models using the plurality of forward models and an ensemble of reverse models using the plurality of reverse models **340**

Identifying a best forward model and a best reverse model using the model evaluation score **342**

Choosing a final single model as the ensemble classification model from amongst the best forward models and the best reverse model, and a final ensemble classification model from among the ensemble of forward models and the ensemble of reverse models, based on the classification of the individual training biological samples from the training data **344**

FIG. 3C

Saliva sample

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321028613